(19) Europäisches Patentamt
European Patent Office
Office européen des brevets

(11) **EP 3 409 357 B1**

(12) **EUROPEAN PATENT SPECIFICATION**

(45) Date of publication and mention
of the grant of the patent:
**01.06.2022 Bulletin 2022/22**

(21) Application number: **17744139.1**

(22) Date of filing: **23.01.2017**

(51) International Patent Classification (IPC):
**B01J 23/887** *(2006.01)*   **B01J 37/00** *(2006.01)*
**C07C 253/26** *(2006.01)*   **C07C 255/08** *(2006.01)*
**C07B 61/00** *(2006.01)*

(52) Cooperative Patent Classification (CPC):
(C-Sets available)
**B01J 23/8878; B01J 23/8876; B01J 35/002;**
**B01J 37/0045; B01J 37/08; C07C 253/26;**
B01J 21/08; B01J 2523/00; C07B 61/00;
Y02P 20/52                                    (Cont.)

(86) International application number:
**PCT/JP2017/002144**

(87) International publication number:
**WO 2017/130909 (03.08.2017 Gazette 2017/31)**

(54) **FLUID BED AMMOXIDATION REACTION CATALYST, AND ACRYLONITRILE PRODUCTION METHOD**

WIRBELBETT-AMMOXIDATIONSKATALYSATOR UND
ACRYLNITRILHERSTELLUNGSVERFAHREN

CATALYSEUR DE RÉACTION D'AMMOXYDATION EN LIT FLUIDISÉ, ET PROCÉDÉ DE
PRODUCTION D'ACRYLONITRILE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.01.2016 JP 2016011813**

(43) Date of publication of application:
**05.12.2018 Bulletin 2018/49**

(73) Proprietor: **Asahi Kasei Kabushiki Kaisha
Tokyo 100-0006 (JP)**

(72) Inventors:
• **FUKUZAWA, Akiyoshi
  Tokyo 101-8101 (JP)**
• **KANETA, Masatoshi
  Tokyo 101-8101 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
WO-A1-2017/217343    JP-A- H07 289 901
JP-A- 2004 105 951    JP-A- 2006 341 179
JP-A- 2010 172 851    JP-A- 2015 188 802
JP-A- 2016 120 468    JP-B2- 5 188 005
US-A1- 2006 155 139    US-A1- 2006 194 693

Remarks:
The file contains technical information submitted after
the application was filed and not included in this
specification

EP 3 409 357 B1

(52) Cooperative Patent Classification (CPC): (Cont.)

C-Sets
**C07C 253/26;**
B01J 2523/00

**Description**

Technical Field

The present invention relates to a catalyst for a fluidized bed ammoxidation reaction and a method for producing acrylonitrile using the catalyst for the fluidized bed ammoxidation reaction.

Background Art

A method for producing acrylonitrile by reacting propylene with molecular oxygen and ammonia (ammoxidation reaction) is well-known. Moreover, a large number of catalysts for use in the ammoxidation reaction are proposed.
Various elements are applied to the catalysts for use in producing acrylonitrile, and the number of components given as examples are large. With respect to such multi-elemental composite oxide catalysts, improvements in combinations of components and in compositions have been investigated. For example, Patent Literature 1 discloses a granular porous ammoxidation catalyst containing molybdenum, bismuth, and iron as essential elements and further containing elements such as nickel, cobalt, zinc, magnesium, chromium, potassium, cerium, magnesium, and rubidium as optional components, wherein a starting material for silica, the starting material to be used for producing silica as a carrier for the catalyst, contains a mixture of 40 to 100% by weight of silica sol having an average particle diameter of primary particles of 20 to less than 55 nm and 60 to 0% by weight of silica sol having an average particle diameter of primary particles of 5 to less than 20 nm, the mixture having a standard deviation of 30% or more based on an average diameter in a particle diameter distribution of the primary particles of the silica sol.
In addition, Patent Literature 2 describes a catalyst containing rubidium, cerium, chromium, magnesium, iron, bismuth, and molybdenum as essential elements and further containing nickel or at least one of nickel and cobalt. Further, Patent Literature 3 describes a catalyst using molybdenum, bismuth, iron, chromium, cerium, nickel, magnesium, cobalt, manganese, potassium, and rubidium as essential elements and further using other optional elements.
Furthermore, Patent Literatures 4 and 5 each describe a catalyst in which kinds of elements and concentration ranges of components are specified by a predetermined formula, and besides, the atomic ratios of those elements satisfy a predetermined relationship.
In addition to the above-described catalysts, catalysts specified by the physical properties or the crystallinity thereof have been described in literatures in recent years, and methods for obtaining a highperformance catalyst by a predetermined production method have been proposed. In more detail, a catalyst characterized by the size and ratio of a metal oxide particle in a catalyst particle (see, for example, Patent Literature 6), a catalyst into which an alkali metal is introduced by impregnation operation (see, for example, Patent Literature 7), a catalyst composition in which the ratio of X-ray diffraction peaks is specified (see, for example, Patent Literature 8), and the like have been proposed, and it is described in the literatures that the effect thereof is improvement in the yield of acrylonitrile as an aimed product. JP 2010 172851 A discloses a fluid bed ammoxidation catalyst.

Citation List

Patent Literature

Patent Literature 1: Japanese Patent No. 5188005
Patent Literature 2: Japanese Patent No. 4709549
Patent Literature 3: Japanese Patent No.4588533
Patent Literature 4: Japanese Patent No. 5011167
Patent Literature 5: Japanese Patent No.5491037
Patent Literature 6: Japanese Patent Application Laid-Open No. 2013-17917
Patent Literature 7: Japanese Patent Application Laid-Open No. 2013-169482
Patent Literature 8: National Publication of International Patent Application No. 2013-522038

Summary of Invention

Technical Problem

The yield of acrylonitrile obtained by using any of the catalysts described in the literatures appears to be improved when compared to the yields of acrylonitrile in the past; however, as is clear from the descriptions of these literatures, byproducts in the reaction still exist. Thus, a further improvement in the yield of acrylonitrile as an aimed product is desired. Moreover, when the byproducts of the reaction are decreased, load relating to separation, collection, and disposal of the aimed product and the byproducts are decreased, so that an environmental effect is also expected. Therefore, a method for further decreasing the byproducts in producing acrylonitrile without relying upon conventional techniques, namely a method and a catalyst which are capable of achieving a high yield have been required. Moreover, it is important in order to achieve the high yield that the consumption of starting materials be decreased, and a high yield can be maintained stably over a long time. In view of the foregoing, a catalyst, which has excellent practicability/handling properties with respect to life performance, shape and strength, is required.

To obtain acrylonitrile at a high yield as described above, it is possible to improve the yield of the aimed product by increasing the molar ratio of ammonia to propylene in starting materials. On the other hand, ammonia in the starting materials is consumed in the nitrilization of propylene in the starting materials, and besides, the ammonia may be converted to nitrogen by oxidative decomposition or may be left as an unreacted component without being consumed in the reaction. Therefore, when unreacted ammonia is left in a relatively large amount, namely, when ammonia is excessively used, the yield of the aimed product is increased. In such a condition, however, a large amount of sulfuric acid for disposing of unreacted ammonia may become necessary or the disposal of ammonium sulfate produced through the disposal with sulfuric acid may further become necessary. Accordingly, a catalyst is desirable which provides a good yield even when unreacted ammonia is not left so much, namely, even when an excessive amount of ammonia is not used. It can be said that the performance of each of the catalysts described in Patent Literatures 1 to 8 is still insufficient in performance from the viewpoint described above.

The present invention has been completed in view of the problem of the conventional techniques, and an object of the present invention is to obtain a catalyst that is capable of producing acrylonitrile at a high yield and stably over a long time even under conditions in which an excess amount of ammonia is not used in producing acrylonitrile through ammoxidation reaction of propylene.

Solution to Problem

The present inventors have conducted diligent studies in order to solve the problem to finally find out that as a catalyst for use in producing acrylonitrile by reacting propylene with molecular oxygen and ammonia, the catalyst described below exhibits a high reaction yield of acrylonitrile even under conditions where an excessive amount of ammonia is not used and also exhibits excellent performance in terms of handling properties, and have completed the present invention.

That is, the present invention is as follows.

[1] A catalyst for a fluidized bed ammoxidation reaction comprising:

silica; and
a metal oxide, wherein
a composite of the silica and the metal oxide is represented by the following formula (1):

$$Mo_{12}Bi_aFe_bNi_cMg_dCo_eCe_fCr_gZn_hX_iO_j/(SiO_2)_A \quad \cdots\cdots\cdots\cdots (1);$$

wherein Mo represents molybdenum, Bi represents bismuth, Fe represents iron, Ni represents nickel, Mg represents magnesium, Co represents cobalt, Ce represents cerium, Cr represents chromium, Zn represents zinc, X represents at least one element selected from the group consisting of potassium, rubidium, and cesium, $SiO_2$ represents silica, a, b, c, d, e, f, g, h, i, and j each represent an atomic ratio of each element and satisfy $0.1 \leq a \leq 1$, $0.5 \leq b \leq 4$, $0.5 \leq c \leq 6.5$, $0.2 \leq d \leq 2.5$, $0.5 \leq e \leq 7$, $0.4 \leq f \leq 1.2$, $g \leq 0.05$, $h \leq 4$, and $0.05 \leq i \leq 1$, provided that j represents an atomic ratio of an oxygen atom, the atomic ratio satisfying valences of constituent elements excluding silica, A represents a content of silica (% by mass) in the composite and satisfies $35 \leq A \leq 48$, and values of $\alpha$, $\beta$, and $\gamma$ calculated from the atomic ratios of respective elements by the following expressions (2), (3), and (4) satisfy $0.03 \leq \alpha \leq 0.12$, $0.1 \leq \beta \leq 0.7$, and $0.2 < \gamma < 4$:

$$\alpha = 1.5a/(1.5(b + g) + c + d + e + h) \quad \cdots\cdots\cdots\cdots (2);$$

$$\beta = 1.5(b + g)/(c + d + e + h) \quad \cdots\cdots\cdots\cdots\cdots (3);$$

and

$$\gamma = e/(c + d + h) \quad \cdots\cdots\cdots\cdots\cdots\cdots\cdots\cdots\cdots\cdots\cdots\cdots\cdots \quad (4).$$

[2] The catalyst for the fluidized bed ammoxidation reaction according to [1], wherein $\delta$ calculated from the atomic ratio of each element by the following expression (5) satisfies $-1 \leq \delta \leq 1.5$:

$$\delta = 12 - 1.5(a + b + f + g) - c - d - e - h \quad \cdots \quad (5).$$

[3] The catalyst for the fluidized bed ammoxidation reaction according to [1] or [2], wherein the X represents rubidium.
[4] The catalyst for the fluidized bed ammoxidation reaction according to any of [1] to [3], wherein $\varepsilon$ calculated from the atomic ratio of each element by the following expression (6) satisfies $0.5 \leq \varepsilon \leq 3.0$:

$$\varepsilon = f/a \quad \cdots\cdots\cdots\cdots\cdots\cdots\cdots\cdots\cdots\cdots\cdots\cdots\cdots\cdots\cdots \quad (6).$$

[5] A method for producing the catalyst for the fluidized bed ammoxidation reaction according to any one of [1] to [4], the method comprising:

a first step of preparing a starting material slurry;
a second step of obtaining a dried particle by spray-drying the starting material slurry; and
a third step of calcining the dried particle, wherein
a silica sol, which has an average particle diameter of primary particles of 5 nm or more and less than 50 nm and has a standard deviation of less than 30% based on the average particle diameter in a particle size distribution of the primary particles, is used as a starting material for the silica in the first step.

[6] A method for producing acrylonitrile using the catalyst for the fluidized bed ammoxidation reaction according to any of [1] to [4].

Advantageous Effects of Invention

When the catalyst for the fluidized bed ammoxidation reaction according to the present invention is used, acrylonitrile can be produced at a high yield and stably over a long time even under conditions in which an excessive amount of ammonia based on propylene is not used in producing acrylonitrile through ammoxidation reaction of propylene.

Description of Embodiments

Hereinafter, an embodiment for carrying out the present invention (hereinafter, simply referred to as "the present embodiment") will be described in detail. The present embodiment below is an example for describing the present invention and does not intend to limit the present invention to the contents below. The present invention can be carried out by making various modifications within a range of the scope thereof.

[Composition of Catalyst]

The catalyst for the fluidized bed ammoxidation reaction according to the present embodiment comprises: silica and a metal oxide, wherein a composite of the silica and the metal oxide is represented by the following formula (1).

$$Mo_{12}Bi_aFe_bNi_eMg_dCo_eCe_fCr_gZn_hX_iO_j/(SiO_2)_A \quad \cdots\cdots\cdots \quad (1);$$

wherein Mo represents molybdenum, Bi represents bismuth, Fe represents iron, Ni represents nickel, Mg represents magnesium, Co represents cobalt, Ce represents cerium, Cr represents chromium, Zn represents zinc, X represents at least one element selected from the group consisting of potassium, rubidium, and cesium, $SiO_2$ represents silica, a, b, c, d, e, f, g, h, i, and j each represent an atomic ratio of each element and satisfy $0.1 \leq a \leq 1$, $0.5 \leq b \leq 4$, $0.5 < c \leq 6.5$, $0.2 \leq d \leq 2.5$, $0.5 \leq e \leq 7$, $0.4 \leq f \leq 1.2$, $g \leq 0.05$, $h \leq 4$, and $0.05 \leq i \leq 1$, provided that j represents an atomic ratio of an oxygen atom, the atomic ratio satisfying valences of constituent elements excluding silica, A represents a content of silica (% by mass) in the composite and satisfies $35 \leq A \leq 48$, and values of $\alpha$, $\beta$, and $\gamma$ calculated from the atomic ratios of respective elements by the following expressions (2), (3), and (4) satisfy $0.03 \leq \alpha \leq 0.12$, $0.1 \leq \beta \leq 0.7$, and $0.2 \leq \gamma \leq 4$:

$$\alpha = 1.5a/(1.5(b + g) + c + d + e + h) \quad \cdots\cdots\cdots (2);$$

$$\beta = 1.5(b + g)/(c + d + e + h) \quad \cdots\cdots\cdots\cdots (3);$$

and

$$\gamma = e/(c + d + h) \quad \cdots\cdots\cdots\cdots\cdots\cdots\cdots\cdots (4).$$

The catalyst for the fluidized bed ammoxidation reaction according to the present embodiment is constituted as described above, and therefore acrylonitrile can be produced at a high yield and stably over a long time under conditions in which an excess amount of ammonia based on propylene is not used in producing acrylonitrile through ammoxidation reaction of propylene.

In the catalyst for the fluidized bed ammoxidation reaction according to the present embodiment, as can be understood from the composition represented by the formula (1), the atomic ratio of Cr, g, is 0.05 or less, the atomic ratio of Zn, h, is 4 or less, and besides, X is at least one element selected from the group consisting of potassium, rubidium, and cesium, the atomic ratio of X is in a range of $0.05 \leq i \leq 1$, and the atomic ratio of any of molybdenum, bismuth, iron, nickel, magnesium, cobalt, and cerium exceeds 0.1. Thus, it can be said that the catalyst comprises a composite obtained by adding silica to a metal oxide comprising at least 8 metal elements.

Each element has its own role for functioning as a catalyst. For example, there exist an element the function of which can substitute for the function of another element, an element the function of which cannot substitute for the function of another element, and an element that brings about an auxiliary action for the function of another element. In view of the foregoing, the preferable element to be appropriately and selectively added, and the composition ratios may vary depending on the elemental constitution and the composition of the catalyst. It is to be noted that the ratio of each element can be specified from the ratio of a starting material for the catalyst charged. Moreover, the elemental composition and the amount of the silica carrier (% by mass) can be confirmed by quantitative analysis measurement with an X-ray fluorescence spectrometer. For example, quantitative analysis of a catalyst sample is performed based on a calibration curve for correcting the matrix effect of each element using a ZSX 100e X-ray fluorescence spectrometer (tube: Rh-4 KW, dispersive crystal: LiF, PET, Ge, RX25) manufactured by Rigaku Corporation. The values obtained by the analysis nearly coincide with the charge composition and the amount of the silica carrier (% by mass) in designing a catalyst. That is, the catalyst composition can be determined not only before the catalyst is used but also when the catalyst is being used by using the measurement method.

Molybdenum is a main element for forming the metal oxide. Molybdenum has a function of making a composite oxide form with another metal element and facilitating ammoxidation reaction, and the content thereof is the largest among the metal elements that constitute the catalyst for the fluidized bed ammoxidation reaction according to the present embodiment.

Bismuth forms a composite oxide with molybdenum, iron, or cerium, and is an element to form an adsorption reaction field for propylene. When the amount of bismuth is small, the adsorption reaction field for propylene is lost, so that the activity of propylene and the yield of acrylonitrile are lowered. In addition, when the amount of bismuth is too large, the decomposition activity of propylene is enhanced to lower selective reaction for acrylonitrile. Therefore, the atomic ratio of bismuth, a, is in a range of $0.1 \leq a \leq 1$ when the atomic ratio of molybdenum is assumed to be 12. From the same viewpoint as described above, the atomic ratio of bismuth, a, is preferably in a range of $0.2 \leq a \leq 0.8$, more preferably in a range of $0.3 \leq a \leq 0.7$.

It is inferred that iron has a function of facilitating the reduction-oxidation ability of the catalyst in the adsorption reaction field for propylene. When propylene is subjected to ammoxidation, lattice oxygen in the catalyst is consumed and the catalyst is subjected to reduction. When such reaction goes on, oxygen in the catalyst is lost, so that ammoxidation reaction does not proceed and the catalyst is deteriorated by reduction. Therefore, a function of taking oxygen in a gas phase into the catalyst to suppress the deterioration of the catalyst by reduction becomes necessary, and it is inferred that iron takes the role. Moreover, when the atomic ratio of iron is small, the function works insufficiently, and when the atomic ratio of iron is large, the oxidation ability of the catalyst is enhanced, so that the decomposition activity of propylene is enhanced to lower acrylonitrile selectivity. From the viewpoint described above, a suitable atomic ratio of iron, b, is in a range of $0.5 \leq b \leq 4$, preferably in a range of $0.8 \leq b \leq 3$, and more preferably in a range of $1 \leq b \leq 2$.

It is inferred that nickel has an action of helping the function of iron. Iron takes a divalent or trivalent form in terms of valency due to the reduction-oxidation action of the catalyst, while nickel exists as a divalent form, and it is inferred that nickel has a function of stabilizing the reduction-oxidation action of the catalyst. From the viewpoint described above, a suitable atomic ratio of nickel, c, is in a range of $0.5 \leq c \leq 6.5$, preferably in a range of $1 \leq c \leq 6$, and more preferably in a range of $1.5 \leq c \leq 5$.

Magnesium has an action of helping the function of nickel and has a role as an element for facilitating stabilization of a crystal structure. The fluidized bed catalyst receives shocks due to the contact and the like with a reactant gas in addition to the collision between the catalysts and the collision with a wall in a reactor. The crystal structure of the catalyst is changed by the shocks and the reactivity may be lowered. Magnesium has a function of hardly causing such a change. Moreover, iron, nickel, and cobalt easily get into the same crystal structure and may cause a structural change in a reaction process, but when magnesium is contained, the structural change is suppressed, so that it is inferred that magnesium also has a function as a stabilizer. From the viewpoint described above, a suitable atomic ratio of magnesium, d, is in a range of $0.2 \leq d \leq 2.5$, preferably in a range of $0.2 \leq d \leq 2$, and more preferably in a range of $0.2 \leq d \leq 1.5$.

It is inferred that cobalt has a function that is similar to that of nickel and of magnesium. The crystal structure of a composite oxide form of nickel and molybdenum (nickel molybdate) is easily changed by shock, but in contrast, the crystal structure of a composite oxide form of cobalt and molybdenum (cobalt molybdate) is stable, and the introduction of cobalt leads to the stabilization of the crystal structure during usage for a long time and the reaction stability is also enhanced. On the other hand, cobalt more easily makes a composite oxide form with iron than nickel does and has a function of suppressing the effective action of iron, and therefore a suitable range for cobalt exists, which is similar to other elements. From the viewpoint described above, the suitable atomic ratio of cobalt, e, is in a range of $0.5 \leq e \leq 7$, preferably in a range of $1 \leq e \leq 7$, and more preferably in a range of $1.5 \leq e \leq 6$.

Cerium has an effect of improving thermal stability. The catalyst is influenced by the reaction temperature during reaction, and when the thermal stability is low, transfer of metal elements occurs inside the catalyst particle, and there is a possibility that the transfer of the metal elements has an influence on and lowers the performance. Particularly, the Tammann temperature of the composite oxide form of bismuth molybdate is low, and the structural stability is low under reaction conditions. It is inferred that cerium in the composite oxide has functions of stabilizing the structure, enhancing the thermal stability, and suppressing the transfer of the metal elements in the catalyst particle. A suitable atomic ratio of cerium, f, is in a range of $0.4 \leq f \leq 1.2$, preferably in a range of $0.4 \leq f \leq 1$, and more preferably in a range of $0.6 \leq f \leq 1$.

It is inferred that chromium is an element that acts on iron. Iron takes a divalent or trivalent form in terms of valence due to the reduction-oxidation action of the catalyst, while chromium exists as a trivalent form, and it is inferred that chromium has a function of suppressing the decomposition activity of iron. When chromium is contained in the catalyst system, $CO_x$ being a decomposition product in reaction decreases and the performance of acrylonitrile selectivity is enhanced. However, when a large amount of chromium is contained, there is a tendency that the function of reduction-oxidation action becomes poor and the activity is gradually lowered during use over a long time. A suitable atomic ratio, g, for achieving the stabilization over a long time from a practical point of view is $g \leq 0.05$, more preferably $g \leq 0.03$. In addition, g=0 is preferable in the case where an improvement of only catalyst life is the object.

Similar to the case with nickel and magnesium, zinc forms a compound with molybdenum. In that case, zinc forms a divalent molybdate in the same manner as in the case of nickel and of magnesium, and therefore the divalent molybdate is incorporated into the structure of nickel molybdate and of magnesium molybdate depending on the amount handled. This action gives an influence on the characteristics of the structural body and an effect that is good for the reaction characteristics of the catalyst can be exhibited. A suitable atomic ratio of zinc, h, varies according to the amount and ratio of nickel and of magnesium but is $h \leq 4$ based on 12 of molybdenum. In addition, the effect of zinc may be obtained insufficiently depending on the atomic ratios of iron and cobalt in addition to nickel and magnesium. In such a case, the desired effects according to the present embodiment are achieved without using zinc, namely with h=0.

An alkali metal being X has a function of controlling propylene activity of the catalyst. A suitable ratio, i, for adjusting the activity varies according to each element such as potassium, rubidium, or cesium, but is generally in a range of $0.05 \leq i \leq 1$. When the amount of the alkali metal is small, the propylene activity is high, but the decomposition activities of propylene and of a product are also enhanced to lower the yield of acrylonitrile. On the other hand, when the amount of the alkali metal is large, there is a tendency that the yield of acrylonitrile is increased, but when the amount of the alkali metal is too large, the propylene activity is lowered, and on top of that, good active sites for producing acrylonitrile are lost, and therefore a suitable amount of the alkali metal needs to be used. From the viewpoint described above, a suitable atomic ratio of X, i, is preferably in a range of $0.05 \leq i \leq 0.7$, more preferably in a range of $0.05 \leq i \leq 0.5$.

Each element in the catalyst for the fluidized bed ammoxidation reaction according to the present embodiment has its own role as described above, and it is considered that there exist an element the function of which can substitute for the function of another element, an element the function of which cannot substitute for the function of another element, an element that brings about an auxiliary action for the function of another element, and the like. By designing a catalyst that can exhibit the functions of respective elements in good balance, an aimed product can be obtained at a high yield and stably over a long time. The relationships satisfied by the parameters represented by the expressions (2), (3), and (4) are obtained by expressing the technical ideas of the present inventors by a numerical expression in designing a catalyst for the purpose of producing the catalyst having desired performance. Specifically, the relationships are based on the technical ideas below.

$\alpha$ represented by the expression (2) denotes a ratio of the element that forms a composite oxide to be the adsorption reaction field for propylene and the elements that form a composite oxide having a role of keeping the reaction stable in the reaction field. When the number of reaction sites is large, the amount of a substance that keeps the reaction stable at the reaction sites increases. Specifically, the relationship between the amount of bismuth molybdate and the amounts of iron, chromium, nickel, cobalt, and zinc is important to produce acrylonitrile at a high yield and stably over a long time in the reaction of propylene, wherein bismuth molybdate mainly functions in the reaction for producing acrylonitrile from propylene, and wherein iron, chromium, nickel, magnesium, cobalt, and zinc have a function of supplying the lattice oxygen, which is in the catalyst and is to be consumed in the reaction, from the molecular oxygen in the gas phase into the catalyst. Thus, $\alpha$ satisfies a condition of $0.03 \leq \alpha \leq 0.12$, preferably satisfies a condition of $0.04 \leq \alpha \leq 0.1$, and more preferably satisfies a condition of $0.05 \leq \alpha \leq 0.08$. When $\alpha$ is less than 0.03, the reaction activity of the catalyst is lowered, and further, the nitrilization reaction becomes hard to progress, and therefore the acrylonitrile selectivity is lowered and the yield of acrylonitrile is lowered. A decrease in the reaction fields brings about the deterioration of performance with time particularly in using the catalyst for a long time. On the other hand, when $\alpha$ exceeds 0.12, the amount of carbon monoxide and carbon dioxide being byproducts in the reaction increases and the yield of acrylonitrile is lowered although the low activity observed when $\alpha$ is low does not occur. From the fact, it is suggested that the decomposition activity of propylene be enhanced.

It is to be noted that multiplying the atomic ratios of bismuth, iron, and chromium by a coefficient of 1.5 is because it is inferred that 1.5 mol of the composite oxide is formed based on 1 mol of molybdenum. On the other hand, nickel, magnesium, cobalt, and zinc form 1 mol of the composite oxide based on 1 mol of molybdenum, and therefore the coefficient is set to 1.

Next, $\beta$ represented by the expression (3) has an influence on the oxidation-reduction action of the catalyst. It is considered that the lattice oxygen in the catalyst is consumed by the reaction, meanwhile the composite oxide form of iron has an effect of incorporating the molecular oxygen in the gas phase into the catalyst. Specifically, it is inferred that iron molybdate containing trivalent iron is reduced into iron molybdate containing divalent iron, or conversely, iron molybdate containing divalent iron is oxidized into iron molybdate containing trivalent iron, thereby keeping the amount of oxygen in the catalyst constant while reduction-oxidation of the catalyst is repeated. It is inferred that chromium exhibits an effect of stabilizing the structure of iron molybdate containing trivalent iron, and a molybdate of nickel, of magnesium, of cobalt, and of zinc act on an effect of stabilizing the structure of iron molybdate containing divalent iron and an effect of helping the function of iron molybdate containing divalent iron. Therefore, the value of $\beta$ denotes balance of elements which adjust the reduction-oxidation action and is an important factor as an effect that is given to the catalyst performance. As a suitable range of $\beta$, $\beta$ satisfies a condition of $0.1 \leq \beta \leq 0.7$, preferably satisfies a condition of $0.2 \leq \beta \leq 0.6$, and more preferably satisfies a condition of $0.2 \leq \beta \leq 0.4$. When $\beta$ is less than 0.1, the activity becomes too low. On the other hand, when $\beta$ exceeds 0.7, the yield of the aimed product is lowered.

Next, $\gamma$ represented by the expression (4) denotes the atomic ratio of cobalt to the total of nickel, magnesium, and zinc. It is inferred that cobalt and nickel have a function of stabilizing the reduction-oxidation action of iron. However, nickel molybdate is weak against shocks in terms of the stability of the crystal structure and has a feature that a change in the crystal occurs due to the shocks, such as collision between catalysts, collision with a wall surface of a reactor, and contact with a gas, which occur in the fluidized bed reaction, and the reaction activity is lowered. Therefore, it is effective to add magnesium that contributes to the stabilization of the crystal structure. Cobalt molybdate has a stronger shock resistance than nickel molybdate and exhibits an effect of the stabilization of the crystal. Moreover, cobalt has a strong interaction with ion and has a function of suppressing the decomposition activity due to iron, and it is suggested that cobalt also have an effect of improving the yield of acrylonitrile. However, an increase in the amount of cobalt allows making solid solution with iron to progress, causing a change with time or suppressing the function of iron too much, so that the deterioration of performance may be brought about. Therefore, it is important to keep balance between the performance and the structural stabilization by adding magnesium to nickel and cobalt. Moreover, zinc also gets into the structure of nickel molybdate and of magnesium molybdate and therefore has relation with the quantity ratio to cobalt. The atomic ratios are important factors for obtaining acrylonitrile at a high yield and stably over a long time. As a suitable range of $\gamma$, $\gamma$ satisfies a condition of $0.2 \leq \gamma \leq 4$, preferably satisfies a condition of $0.3 \leq \gamma \leq 3.5$, and more preferably satisfies a condition of $0.4 \leq \gamma \leq 3.5$.

The catalyst for the fluidized bed ammoxidation reaction according to the present embodiment has, as represented by the above formula (1), a content of silica A (% by mass) of 35 to 48. The mass ratio of silica in the catalyst is not only an important factor for obtaining the shape and strength of the catalyst, which are important for use in the fluidized bed reaction, but also is important as a feature for obtaining a high reactivity. When the amount of silica is small, a spherical particle that is necessary as a fluidized bed catalyst is difficult to form, so that not only the smoothness of the particle surface is lowered, but also the attrition strength and crushing strength are lowered. On the other hand, when the amount of silica is large, the reactivity, such as the yield of acrylonitrile, is lowered. From the viewpoint described above, the A is preferably from 35 to 45, more preferably from 38 to 45, and still more preferably from 38 to 43.

In the catalyst for the fluidized bed ammoxidation reaction according to the present embodiment, δ preferably satisfies a relationship of -1≤δ≤1.5 in the case where the atomic ratios of respective metal elements being components that constitute the catalyst are substituted into the following expression (5).

$$\delta = 12 - 1.5(a + b + f + g) - c - d - e - h \quad \cdots \quad (5)$$

δ denotes an atomic ratio of molybdenum that does not form the composite oxide of molybdenum as molybdenum oxide and is preferably adjusted in the range from the viewpoint of further enhancing the catalyst performance. In the case where δ is -1 or more, there is a tendency that an increase in the decomposition activity attributable to an increase in the amount of metal elements that cannot form the composite oxide with the molybdenum element can be prevented and lowering of the reaction selectivity for an aimed product can be prevented effectively. On the other hand, in the case where δ is 1.5 or less, there is a tendency that a problem on handling properties such that the amount of molybdenum oxide is increased to deteriorate the catalyst shape, or the molybdenum oxide precipitates from the catalyst particle during reaction to deteriorate the fluidity can be prevented effectively. From the viewpoint of making both the reactivity and the handling properties satisfactory in this way, the δ calculated by the expression (5) preferably satisfies a relationship of -1≤δ≤1.5, and more preferably satisfies a relationship of -0.5≤δ≤1. Thereby, the catalyst becomes excellent in industrial practicability because the attrition strength and the handling properties such as the particle shape become further satisfactory, and there is a tendency that the stability of the reaction performance can be maintained for a further long time.

In the catalyst for the fluidized bed ammoxidation reaction according to the present embodiment, potassium, rubidium, and cesium can be contained as alkali metals, but among them, rubidium is preferable. The degree of influence on the activity becomes larger in the order of potassium, rubidium, and cesium, and the amount to be used becomes smaller by changing from potassium to rubidium and to cesium. It is to be noted that changes in the acrylonitrile selectivity are observed depending on the kind of the alkali metal, and there is a tendency that cesium is superior in the acrylonitrile selectivity to potassium, and rubidium is superior in the acrylonitrile selectivity to cesium. A suitable atomic ratio of rubidium, i, in the case where rubidium is used as X is preferably in a range from 0.05 to 0.3, more preferably in a range from 0.1 to 0.25.

The catalyst for the fluidized bed ammoxidation reaction according to the present embodiment preferably satisfies a relationship of 0.5≤ε≤3.0 in the case where the values of the atomic ratio of bismuth, a, and the atomic ratio of cerium, f, are substituted into the following expression (6).

$$\varepsilon = f/a \quad \cdots\cdots\cdots\cdots\cdots\cdots\cdots\cdots\cdots\cdots\cdots\cdots\cdots\cdots \quad (6)$$

The relationship between the atomic ratio of bismuth, a, and the atomic ratio of cerium, f, has an influence on the yield of acrylonitrile and the life performance of the catalyst. Bismuth forms a composite oxide with molybdenum, iron, or cerium, and is an element to form the adsorption reaction field for propylene. When the amount of bismuth is small, the adsorption reaction field for propylene is lost so that the activity of propylene and the yield of acrylonitrile are lowered, and therefore bismuth is an essential element for obtaining acrylonitrile at a high yield. On the other hand, it is known that the composite oxide of bismuth and molybdenum has a low thermal stability among the composite oxides that are used for favorable reaction. The low thermal stability leads to a change in the catalyst performance because in the reaction for a long time, the change in the crystal structure of the composite oxide occurs, and the transfer of elements occurs in the catalyst particle. Therefore, the stabilization of the crystal structures in the catalyst is preferably achieved for obtaining a catalyst that is stable for a long time. Cerium has a function of acting on the composite oxide form of bismuth and molybdenum to enhance the thermal stability. On the other hand, cerium has a decomposition activity for propylene, and when cerium is contained excessively, the acrylonitrile selectivity may be lowered. That is, the atomic ratio of bismuth and cerium, ε, is preferably adjusted from the viewpoint of obtaining acrylonitrile at a high yield with the catalyst the thermal stability of which is enhanced. In the case where the atomic ratio of bismuth and cerium, ε, is 0.5 or more, a sufficient thermal stability can be secured, so that there is a tendency that the life performance of the catalyst is further improved, and in the case where the atomic ratio is 3 or less, there is a tendency that the yield of acrylonitrile is further improved. From the viewpoint described above, ε more preferably satisfies a relationship of 0.8≤ε≤2.5 and still more preferably satisfies a relationship of 1.5≤ε≤2.5.

[Physical Characteristics of Catalyst]

The catalyst for the fluidized bed ammoxidation reaction according to the present embodiment is used for fluidized bed ammoxidation reaction. In the present embodiment, the shape of the catalyst is preferably a spherical shape. In addition, the average particle diameter of the catalyst particle is preferably in a range from 40 to 70 $\mu$m, more

preferably in a range from 45 to 65 $\mu$m. As the particle diameter distribution, the amount of the catalyst particle having a particle diameter of 5 to 200 $\mu$m is preferably 90 to 100% by mass based on the total mass of the catalyst. Moreover, the volume ratio of the particles of 45 $\mu$m or less per volume of the whole particles is preferably in a range from 10 to 50%, more preferably in a range from 15 to 35%.

The catalyst for the fluidized bed ammoxidation reaction according to the present embodiment preferably has an excellent attrition strength as a fluidized bed catalyst. The fluidized bed catalyst preferably has attrition strength with which the catalyst particle is neither worn nor fractured by the shocks such as contact between catalysts in a reactor, collision with a wall surface of a reactor, and contact with a gas, and preferably has a attrition loss of 2.5% or less in a attrition test for a fluidized bed catalyst (test according to method described in "Test Method for Synthetic Fluid Cracking Catalyst" (American Cyanamid Co. Ltd. 6/31-4m-1/57)). The attrition loss can be measured by the method described in Examples, which will be described later.

Further, the catalyst for the fluidized bed ammoxidation reaction according to the present embodiment preferably has an apparent specific gravity in a range from 0.85 to 1.15 g/cc, more preferably in a range from 0.96 to 1.10, and most preferably in a range from 0.98 to 1.05. In the case where the catalyst has an apparent specific gravity of 0.85 g/cc or more, the influence of the bulk of the catalyst when the catalyst is put into a reactor can be decreased, so that there is a tendency that the volume of the reactor to be required can be decreased, and besides, there is a tendency that the occurrence of catalyst loss caused by an increase in the amount of the catalyst scattering from the reactor to the outside can be prevented effectively. In addition, in the case where the catalyst has an apparent specific gravity of 1.15 g/cc or less, a satisfactory flow state of the catalyst can be secured, so that there is a tendency that lowering of the reaction performance can be prevented effectively. The apparent specific gravity can be measured by the method described in Examples, which will be described later.

[Method for Producing Catalyst]

The method for producing the catalyst for the fluidized bed ammoxidation reaction according to the present embodiment is not particularly limited, and the catalyst for the fluidized bed ammoxidation reaction according to the present embodiment can be produced by a publicly known method, for example, by a method including: a first step of preparing a starting material slurry; a second step of obtaining a dried particle by spray-drying the starting material slurry; and a third step of calcining the dried particle obtained in the second step.

In the first step, starting materials for the catalyst are blended to obtain a starting material slurry. The source of each element of molybdenum, bismuth, iron, nickel, magnesium, cobalt, cerium, chromium, zinc, potassium, rubidium, and cesium is not particularly limited, and examples thereof include ammonium salts, nitrates, hydrochlorides, sulfates, and organic acid salts which are soluble in water or nitric acid. As the starting material for molybdenum, ammonium salts are preferable, and as the source of each element of bismuth, iron, nickel, magnesium, cobalt, cerium, chromium, zinc, potassium, rubidium, and cesium, nitrates are preferable.

On the other hand, as a starting material for a carrier, silica sol can be used. Various kinds of silica sol in terms of the purity, the amount of impurities, the pH, the particle diameter, and the like can be used, and two or more kinds of silica sol, not limited to one kind of silica sol, can be mixed for use. In the case of silica sol that contains aluminum as an impurity among various kinds of silica sol, the silica sol that contains 0.04 atoms or less of aluminum per 100 atoms of silicon, more preferably 0.02 atoms or less of aluminum per 100 atoms of silicon is used. The pH of silica has an influence on the viscosity of the starting material slurry and therefore may be selected appropriately according to the amount and pH of metal salts to be used.

In addition, with respect to the particle diameter of the silica sol, the silica sol having an average particle diameter of primary particles in a range from 5 to 50 nm is preferably used, and the silica sol further having a standard deviation of less than 30% based on the average particle diameter in the particle diameter distribution of the primary particles is preferably used.

In the present embodiment, by appropriately mixing a plurality of silica sol in which the average diameter and particle diameter distribution of primary particles are known, or other methods, the average particle diameter and standard deviation of silica sol to be obtained can be adjusted to be in the range.

When the primary particles of the silica sol are controlled, the specific surface area, the pore volume, the pore distribution of the catalyst for the fluidized bed ammoxidation reaction are made suitable, and as a result, there is a tendency that the reactivity is improved. Further, when the influence on the strength and the practicability of the catalyst for the fluidized bed ammoxidation reaction is taken into account, the silica sol having an average particle diameter of primary particles of 8 to 35 nm and having a standard deviation of less than 30% based on the average particle diameter in the particle diameter distribution of the primary particles is more preferably used.

In the conventional techniques, it has been known that when the average particle diameter of primary particles of silica sol is too small, the reactivity of a catalyst is lowered, and when the average particle diameter of primary particles of silica sol is too large, the practicability, such as fracture strength, the compressive strength, and the attrition resistance of the catalyst are lowered. Therefore, in Patent Literature 1 for example, mixing of silica sol having an average particle diameter of 20 to 50 nm and silica sol having a smaller average particle diameter, as small as from 5 to 20 nm, are proposed.

When the two kinds of silica sol each having a different average particle diameter are mixed in this way, the standard deviation based on the average particle diameter in the particle diameter distribution of primary particles becomes large. However, the standard deviation based on the average particle diameter in the particle diameter distribution of primary particles of silica gives an influence on the constitution of the metal oxide and silica each existing in the catalyst particle, and a smaller standard deviation ought to be preferable under normal circumstances because when the standard deviation is too large, the reaction stability and the structural stability during usage for a long time are lowered. In the catalyst for the fluidized bed ammoxidation reaction according to the present embodiment, lowering of the reactivity hardly occurs even in the case where the average particle diameter of the primary particles of the silica sol is small by making the catalyst composition as described above. Accordingly, the apparent specific gravity can be increased to improve attrition strength without sacrificing the reactivity, and further, the structural stability during usage for a long time can be enhanced by using the silica sol having a small average particle diameter and having a small standard deviation in the catalyst for the fluidized bed ammoxidation reaction having the above-described composition.

It is to be noted that the average diameter of the primary particles of the silica sol can be determined by a BET method, an electron microscopic method, or the like, and the particle diameter distribution of the primary particles of the silica sol can be determined by a publicly known method such as an electron microscopic method.

It is to be noted that the above-described numerical value of the average diameter of the primary particles in the present embodiment is determined by a BET adsorption isotherm (Brunauer-Emment-Teller adsorption isotherm). Specifically, in the case of the silica sol, water being a dispersion medium for the sol is evaporated at a temperature of 100 to 200°C to obtain a powder, nitrogen is then adsorbed to reach saturation at the liquid nitrogen temperature, and the powder is brought back to room temperature to calculate the specific surface area S ($m^2/g$) of the powder from the amount of nitrogen desorbed. The diameter D (nm) can be determined by the following expression where all the primary particles of the silica sol are assumed to have a spherical shape having the same diameter D (nm), the specific gravity ($\rho$) of the silica particle (amorphous silica) in the silica sol is assumed to be 2.2, and the number of silica particles per 1 g is represented by n.

$$1/\rho = 4/3 \times \pi \times (D \times 10^{-7}/2)^3 \times n$$

$$S = 4 \times \pi \times (D \times 10^{-9}/2)^2 \times n$$

Accordingly, D = 6000/($\rho \times$ S)

In addition, the above-described numerical value of the standard deviation in the particle diameter distribution of the primary particles of the silica sol in the present embodiment is obtained based on the particle diameter distribution determined by the electron microscopic method. Specifically, the diameter and the number of particles having the diameter are determined for respective particles from a photograph of the silica sol taken with an electron microscope to determine the particle diameter distribution statistically. Deviations are calculated from the values of the diameters of individual particles and the average value of the diameters each obtained from the particle diameter distribution to determine the sum total of the squares of the deviations. Dispersion is defined as a value obtained by dividing the sum total by the number of data (number of particles), and the square root of the value is used as the standard deviation.

$$\text{Standard deviation} = \sqrt{(\text{sum of (measured value of each particle diameter} - \text{average particle diameter})^2/\text{number of data})}$$

The standard deviation is a criterion of variation of data, and the larger value means that the data varies more. That is, the larger value means that the shift from the average value is larger. In the present embodiment, a value obtained by dividing the standard deviation by the average particle diameter of the primary particles is expressed by a percentage to use as a criterion of the standard deviation.

The method for blending the starting material slurry is not particularly limited but is, for example, as follows. First, an ammonium salt of molybdenum dissolved in water is added to the silica sol. The slurry can be obtained by subsequently adding thereto a solution obtained by dissolving nitrates each being a source of each element of bismuth, iron, nickel, magnesium, cobalt, cerium, chromium, zinc, potassium, rubidium, cesium, and the like in water or in a nitric acid aqueous solution.

It is to be noted that in the above-described method for blending the starting material slurry, the procedure of adding the sources of respective elements can be changed, or the pH or the viscosity of the slurry can be modified by adjusting the concentration of nitric acid or by adding an ammonia water into the slurry (silica sol). In addition, water-soluble polymers such as polyethylene glycol, methyl cellulose, polyvinyl alcohol, polyacrylic acid, and polyacrylamide, amines, aminocarboxylic acids, multivalent carboxylic acids such as oxalic acid, malonic acid, and succinic acid, and organic acids such as glycolic acid, malic acid, tartaric acid, and citric acid can also be added appropriately. Among others, any of the organic acids having an effect of improving the propylene activity and improving the acrylonitrile yield is preferably added, and oxalic acid or tartaric acid is more preferably added. As the procedure of adding the organic acid described above, the organic acid can be added to the silica sol or to the nitric acid aqueous solution. Moreover, the organic acid can be added to the aqueous solution of molybdenum or to the final slurry. Among others, the organic acid is preferably added to the silica sol.

Next, in the second step, the starting material slurry obtained in the first step is spray-dried to obtain a dried particle having a spherical shape, the dried particle being a catalyst precursor. The method of spraying the starting material slurry is not particularly limited, and spraying of the starting material slurry can be performed by a method such as, for example, a centrifugal system that is usually and industrially carried out, a two-fluid nozzle system, or a highpressure nozzle system, and is preferably performed by a centrifugal system. The source of heat for spray drying is not particularly limited, and examples thereof include steam and an electric heater. The temperature at an inlet of a dryer using air that is heated by the source of heat is preferably from 100 to 400°C, more preferably from 150 to 300°C.

In the third step, the dried particle obtained in the second step is calcined to obtain a desired catalyst composition. If necessary, in the third step, the dried particle is preliminarily calcined, for example, at 150 to 500°C and is then finally calcined in a temperature range from preferably 500 to 730°C, more preferably 550 to 730°C for 1 to 20 hours. The calcination can be performed using a calcining furnace such as a rotary furnace, a tunnel furnace, or a muffle furnace.

[Method for Producing Acrylonitrile]

The method for producing acrylonitrile according to the present embodiment uses the catalyst for the fluidized bed ammoxidation reaction according to the present embodiment. That is, acrylonitrile can be produced by subjecting propylene, molecular oxygen, and ammonia to ammoxidation reaction in the presence of the catalyst for the fluidized bed ammoxidation reaction according to the present embodiment. The method for producing acrylonitrile according to the present embodiment is carried out in a fluidized bed reactor that is usually used. The purities of propylene and ammonia being starting materials are not necessary high, and propylene and ammonia of industrial grade can be used. In addition, as the molecular oxygen, it is preferable that air be usually used, but a gas in which the oxygen concentration is increased by mixing oxygen with air, or by other methods can also be used.

With respect to the composition of the starting material gas in the case where the source of the molecular oxygen in the method for producing acrylonitrile according to the present embodiment is air, the molar ratio of ammonia and of air to propylene expressed by a ratio of propylene/ammonia/air are preferably in a range of 1/(0.8 to 1.4)/(7 to 12), more preferably in a range of 1/(0.9 to 1.3)/(8 to 11).

The reaction temperature in the method for producing acrylonitrile according to the present embodiment is preferably in a range from 350 to 550°C, more preferably in a range from 400 to 500°C. The reaction pressure is preferably in a range from slightly reduced pressure to 0.3 MPa. The contact time between the starting material gas and the catalyst is preferably from 0.5 to 20 (sec·g/cc), more preferably from 1 to 10 (sec·g/cc).

Examples

Hereinafter, the present embodiment will be described more specifically giving Examples, but the present embodiment is not limited to these Examples. In addition, the method for evaluating various physical properties are as described below.

(Evaluation of Reactivity of Catalyst)

As a reaction apparatus, a fluidized bed reaction tube made of Pyrex (R) glass having an external diameter of 23 mm was used. The reaction temperature T was set to 430°C, the reaction pressure P was set to 0.17 MPa, the amount of the catalyst filled W was set to 40 to 60 g, and the total amount of gas supplied F was set to a 250 to 450 cc/sec (in terms of NTP) condition. In addition, the composition of the supplied starting material gas expressed by a molar ratio of propylene/ammonia/air was set to 1/(0.7 to 1.4)/(8.0 to 13), and the reaction was carried out by appropriately changing the supplied gas composition in the range so that the unreacted ammonia concentration in the reaction gas was around 0.5%, and the unreacted oxygen concentration in the reaction gas was 0.5% or lower. The composition of the starting material gas is shown in Table 2. The reaction gas at several hours after the inception of supplying the starting material was analyzed by gas chromatography using GC-14B manufactured by SHIMADZU CORPORATION to evaluate the initial reaction performance and the life performance of the catalyst. It is to be noted that in Examples and Comparative Examples, the contact time; and the conversion ratio (%), the selectivity, and the yield each used for showing the reaction results are the values defined by the following expressions.

$$\text{Contact time (sec·g/cc)} = (W/F) \times 273/(273 + T) \times P/0.10$$

$$\text{Conversion ratio of propylene (\%)} = M/L \times 100$$

$$\text{Acrylonitrile selectivity (\%)} = N/M \times 100$$

$$\text{Yield of acrylonitrile (\%)} = N/L \times 100$$

In the expressions, L represents a number of moles of propylene supplied, M represents a number of moles of propylene reacted, N represents a number of moles of acrylonitrile produced, and the number of moles of propylene reacted was determined as the number of moles obtained by subtracting the number of moles of propylene in the reaction gas from the number of moles of propylene supplied.

(Shape Observation)

The shape of the catalyst was observed using 3D Real Surface View Microscope VE-9800S manufactured by KEYENCE CORPORATION.

(Measurement of Particle Diameter of Catalyst)

The particle diameter of the catalyst was measured using a laser diffraction/scattering type particle size distribution measuring apparatus LA-300 manufactured by HORIBA, Ltd.

(Measurement of Attrition strength)

Measurement of the attrition strength (attrition strength) of the catalyst as attrition loss was performed in accordance with the method described in "Test Method for Synthetic Fluid Cracking Catalyst" (American Cyanamid Co. Ltd. 6/31-4m-1/57) (hereinafter, referred to as "ACC method").
The attrition strength is evaluated by the attrition loss, and the attrition loss was determined as a value defined as described below.

$$\text{Attrition loss (\%)} = R/(S - Q) \times 100$$

In the expression, Q represents the mass (g) of the catalyst scattering due to wear to the outside between 0 to 5 hours, and R usually represents the mass (g) of the catalyst scattering due to wear to the outside between 5 to 20 hours. S represents the mass (g) of the catalyst provided for the test.

(Measurement of Crystal after Attrition Resistance Test)

The catalyst sample after measuring the attrition strength was measured using a multipurpose X-ray diffractometer D8 ADVANCE manufactured by Bruker Corporation. The strength of the diffraction peak (h) at $2\theta = 13.2 \pm 0.2°$ of the $\beta$ type crystal phase of nickel molybdate and the strength of the diffraction peak (h) at $2\theta = 14.3 \pm 0.2°$ of the $\alpha$ type crystal phase of nickel molybdate were measured to observe whether a change into the $\alpha$ type crystal phase had occurred or not.

(Measurement of Apparent Specific Gravity)

A sieved catalyst was dropped into a 100-cc measuring cup container through a funnel using a powder tester manufactured by HOSOKAWA MICRON KK, and when the container was filled, the surface was leveled off so as not to give vibration and the catalyst was weighed to determine the apparent specific gravity from the calculation of mass/volume (g/cc).

(Measurement of Average Particle Diameter of Silica Sol)

The average particle diameter was determined using an automatic specific surface area measuring apparatus Gemini V2380 manufactured by SHIMADZU CORPORATION from a measured value of the specific surface area of silica sol on which drying treatment was performed. That is, water being a dispersion medium for the silica sol was evaporated at a temperature of 100 to 200°C to obtain a powder, nitrogen was then adsorbed to reach saturation at the liquid nitrogen temperature, and the powder was brought back to room temperature to calculate the specific surface area $S$ ($m^2$/g) of the powder from the amount of nitrogen desorbed. Further, the diameter D (nm) was determined by the following expression where all the primary particles of the silica sol were assumed to have a spherical shape having the same diameter D (nm), the specific gravity (p) of the silica particle (amorphous silica) in the silica sol was assumed to be 2.2, and the number of silica particles per 1 g was represented by n.

$$1/\rho = 4/3 \times \pi \times (D \times 10^{-7}/2)^3 \times n$$

$$S = 4 \times \pi \times (D \times 10^{-9}/2)^2 \times n$$

Accordingly, D = 6000/($\rho \times$ S) (Measurement of Standard Deviation in Particle Distribution of Silica Sol)
Electron microscopic observation of a sample obtained by dropping and then drying silica sol diluted with purified water on a grid with a carbon support film adhered thereto was performed using a field emission type transmission electron microscope HF-2000 manufactured by Hitachi, Ltd. The particle diameter distribution and the standard deviation were determined by taking 3 photographs in which visual fields were changed at 200000 magnifications and measuring the particle diameters of about 3000 silica sol particles in the photographs. That is, deviations were calculated from the values of the diameters of individual particles and the average value of the diameters in the particle diameter distribution obtained statistically to determine the sum total of the squares of the deviations. Dispersion is defined as a value obtained by dividing the sum total by the number of data (number of particles), and the square root of the value was used as the standard deviation.

$$\text{Standard deviation} = \sqrt{(\text{sum of (measured value of each particle diameter} - \text{average particle diameter})^2/\text{number of data})}$$

The standard deviation is a criterion of variation of data, and the larger value means that the data varies more, namely means that the shift from the average value is larger. A value obtained by dividing the standard deviation by the average particle diameter of the primary particles was expressed by a percentage to use as a criterion of the standard deviation.

[Example 1] (not according to the presently claimed invention)

A catalyst represented by the atomic ratios of respective elements and the mass of silica described in Examples 1, 2 in Table 1 was prepared in the manner as described below.
A mixed liquid of two kinds of silica sol was obtained by mixing 666.7 g of aqueous silica sol containing 30% by mass of $SiO_2$ having an average particle diameter of silica primary particles of 12 nm and 500 g of aqueous silica sol containing 40% by mass of $SiO_2$ having an average particle diameter of silica primary particles of 41 nm. It is to be noted that the primary particle diameters and the particle diameter distribution of the starting material obtained by mixing two kinds of silica sol were determined by the BET method and the electron microscopic method to find that the average particle diameter of the primary particles was 18.3 nm, the standard deviation was 7.2 nm, and the ratio of the standard deviation to the average particle diameter was 39%.

Next, 250 g of an 8 wt% oxalic acid aqueous solution was added to the mixed liquid of the silica sol which was under stirring to blend a first mixed liquid. Next, a liquid obtained by dissolving 507.6 g of ammonium paramolybdate [$(NH_4)_6Mo_7O_{24}\cdot4H_2O$] in 906 g of water was added to the first mixed liquid to obtain a second mixed liquid. Subsequently, a liquid obtained by dissolving 29.32 g of bismuth nitrate [$Bi(NO_3)_3\cdot5H_2O$], 146.7 g of iron nitrate [$Fe(NO_3)_3\cdot9H_2O$], 282.4 g of nickel nitrate [$Ni(NO_3)_2\text{-}6H_2O$], 143.1 g of magnesium nitrate [$Mg(NO_3)_2\cdot6H_2O$], 155.5 g of cobalt nitrate [$Co(NO_3)_2\cdot6H_2O$], 31.02 g of cerium nitrate [$Ce(NO_3)_3\cdot6H_2O$], and 4.215 g of rubidium nitrate [$RbNO_3$] in 404 g of nitric acid the concentration of which is 16.6% by mass was added to the second mixed liquid to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle (catalyst precursor) was preliminarily calcined using an electric furnace at 320°C for 2 hours under an air atmosphere and was then finally calcined at 600°C for 2 hours under an air atmosphere (third step) to finally obtain 831 g of a catalyst.

The results that the obtained catalyst had a solid sphere shape, an average particle diameter of 56 $\mu$m, and an apparent specific gravity of 0.97 g/cc were obtained. Next, ammoxidation reaction of propylene was performed using 50 g of the catalyst at a contact time of $\Theta$ = 4.3 (sec·g/cc). The conversion ratio of propylene was 99.2%, the acrylonitrile (written as "AN" in Table 2) selectivity was 85.8%, and the acrylonitrile yield was 85.1% at 24 hours after the start of the reaction.

In addition, the attrition strength was measured for 50 g of the catalyst in accordance with the ACC method to find that the attrition loss (%) was 1.0%.

Further, when the sample after the attrition strength test was analyzed by X-ray diffraction, it was ascertained that the crystal phase of nickel molybdate was the $\beta$ type crystal phase, and the $\alpha$ type crystal phase was hardly present. The catalyst composition (metal composition, the amount of silica ($SiO_2$) carrier, values of $\alpha$, $\beta$, $\gamma$, $\delta$, $\varepsilon$) is shown in Table 1, and the reaction results and the results of physical property measurement are shown in Table 2.

[Example 2] (not according to the presently claimed invention)

The reaction performance after 300 Hr was checked by continuing the ammoxidation reaction of propylene in Example 1 while finely adjusting the contact time so that the conversion ratio of propylene was maintained as it was. The results were that the contact time was $\Theta$ = 4.5 (sec·g/cc), and the acrylonitrile (written as "AN" in Table 2) selectivity was 85.6% and the acrylonitrile yield was 84.8% at a conversion ratio of propylene of 99.1% to find that the reaction performance was stable almost in a range of variation in the analysis. The reaction results are shown in Table 2.

[Example 3]

A catalyst represented by the atomic ratios of respective elements and the mass of silica described in Examples 3, 4 in Table 1 was prepared in the manner as described below.

A mixed liquid of two kinds of silica sol was obtained by mixing 666.7 g of aqueous silica sol containing 30% by mass of $SiO_2$ having an average particle diameter of silica primary particles of 12 nm and 500 g of aqueous silica sol containing 40% by mass of $SiO_2$ having an average particle diameter of silica primary particles of 41 nm. It is to be noted that the primary particle diameters and the particle diameter distribution of the starting material obtained by mixing two kinds of silica sol were determined by the BET method and the electron microscopic method to find that the average particle diameter of the primary particles was 18.7 nm, the standard deviation was 7.5 nm, and the ratio of the standard deviation to the average particle diameter was 40%.

Next, 200 g of a 10 wt% tartaric acid aqueous solution was added to the mixed solution of the silica sol which was under stirring. Next, a liquid obtained by dissolving 499.4 g of ammonium paramolybdate [$(NH_4)_6MO_7O_{24}\cdot4H_2O$] in 892 g of water was added to the first mixed liquid to obtain a second mixed liquid. Subsequently, a liquid obtained by dissolving 51.93 g of bismuth nitrate [$Bi(NO_3)_3\cdot5H_2O$], 134.7 g of iron nitrate [$Fe(NO_3)_3\cdot9H_2O$], 243.1 g of nickel nitrate [$Ni(NO_3)_2\cdot6H_2O$], 140.8 g of magnesium nitrate [$Mg(NO_3)_2\cdot6H_2O$], 173.8 g of cobalt nitrate [$Co(NO_3)_2\cdot6H_2O$], 40.70 g of cerium nitrate [$Ce(NO_3)_3\cdot6H_2O$], and 4.493 g of rubidium nitrate [$RbNO_3$] in 401 g of nitric acid the concentration of which is 16.6% by mass was added to the second mixed liquid to obtain an aqueous raw material mixture (starting material slurry) (first step) .

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 580°C for 2 hours under an air atmosphere (third step) to finally obtain 833 g of a catalyst.

The results that the obtained catalyst had a solid sphere shape, an average particle diameter of 58 $\mu$m, and an apparent specific gravity of 1.00 g/cc were obtained. Next, ammoxidation reaction of propylene was performed using 50 g of the catalyst at a contact time of $\Theta$ = 4.2 (sec·g/cc). The conversion ratio of propylene was 99.3%, the acrylonitrile (written as "AN" in Table 2) selectivity was 85.0%, and the acrylonitrile yield was 84.4% at 24 hours after the start of the reaction.

In addition, the attrition strength was measured for 50 g of the catalyst in accordance with the ACC method to find that the attrition loss (%) was 1.2%.

Further, when the sample after the attrition strength test was analyzed by X-ray diffraction, it was ascertained that the crystal phase of nickel molybdate was the $\beta$ type crystal phase, and the $\alpha$ type crystal phase was hardly present.

The catalyst composition (metal composition, the amount of silica ($SiO_2$) carrier, values of $\alpha$, $\beta$, $\gamma$, $\delta$, $\varepsilon$) is shown in Table 1, and the reaction results and the results of physical property measurement are shown in Table 2.

[Example 4]

The reaction performance after 300 Hr was checked by continuing the ammoxidation reaction of propylene in Example 3 while finely adjusting the contact time so that the conversion ratio of propylene was maintained as it was. The results were that the contact time was $\Theta$ = 4.5 (sec·g/cc), and the acrylonitrile (written as "AN" in Table 2) selectivity was 84.8% and the acrylonitrile yield was 84.2% at a conversion ratio of propylene of 99.3% to find that the reaction performance was stable almost in a range of variation in the analysis. The reaction results are shown in Table 2.

[Example 5]

A catalyst represented by the atomic ratios of respective elements and the mass of silica described in Examples 5, 6 in Table 1 was prepared in the manner as described below.

A mixed liquid of two kinds of silica sol was obtained by mixing 750 g of aqueous silica sol containing 30% by mass of $SiO_2$ having an average particle diameter of silica primary particles of 12 nm and 562.5 g of aqueous silica sol containing 40% by mass of $SiO_2$ having an average particle diameter of silica primary particles of 41 nm. It is to be noted that the primary particle diameters and the particle diameter distribution of the starting material obtained by mixing two kinds of silica sol were determined by the BET method and the electron microscopic method to find that the average particle diameter of the primary particles was 18.2 nm, the standard deviation was 7.1 nm, and the ratio of the standard deviation to the average particle diameter was 39%.

Next, 250 g of an 8 wt% oxalic acid aqueous solution was added to the mixed liquid of the silica sol which was under stirring. Further, a liquid obtained by dissolving 453.2 g of ammonium paramolybdate [$(NH_4)_6Mo_7O_{24}\cdot4H_2O$] in 810 g of water was added to the first mixed liquid to obtain a second mixed liquid. Subsequently, a liquid obtained by dissolving 73.30 g of bismuth nitrate [$Bi(NO_3)_3\cdot5H_2O$], 78.57 g of iron nitrate [$Fe(NO_3)_3\cdot9H_2O$], 63.03 g of nickel nitrate [Ni $(NO_3)_2\cdot6H_2O$], 127.8 g of magnesium nitrate [$Mg(NO_3)_2\cdot6H_2O$], 315.5 g of cobalt nitrate [$Co(NO_3)_2\cdot6H_2O$], 41.55 g of cerium nitrate [Ce $(NO_3)_3\cdot6H_2O$], and 3.763 g of rubidium nitrate [$RbNO_3$] in 395.4 g of nitric acid the concentration of which is 16.6% by mass was added to the second mixed liquid to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 610°C for 2 hours under an air atmosphere (third step) to finally obtain 826 g of a catalyst.

The results that the obtained catalyst had a solid sphere shape, an average particle diameter of 54 $\mu$m, and an apparent specific gravity of 0.98 g/cc were obtained. Next, ammoxidation reaction of propylene was performed using 50 g of the catalyst at a contact time of $\Theta$ = 3.6 (sec·g/cc). The conversion ratio of propylene was 99.1%, the acrylonitrile (written as "AN" in Table 2) selectivity was 85.6%, and the acrylonitrile yield was 84.8% at 24 hours after the start of the reaction.

In addition, the attrition strength was measured for 50 g of the catalyst in accordance with the ACC method to find that the attrition loss (%) showed a low value, as low as 0.4%.

Further, when the sample after the attrition strength test was analyzed by X-ray diffraction, it was ascertained that the crystal phase of nickel molybdate was the $\beta$ type crystal phase, and the $\alpha$ type crystal phase was hardly present.

The catalyst composition (metal composition, the amount of silica ($SiO_2$) carrier, values of $\alpha$, $\beta$, $\gamma$, $\delta$, $\varepsilon$) is shown in Table 1, and the reaction results and the results of physical property measurement are shown in Table 2.

[Example 6]

The reaction performance after 300 Hr was checked by continuing the ammoxidation reaction of propylene in Example 5 while finely adjusting the contact time so that the conversion ratio of propylene was maintained as it was to find that the contact time was almost the same at $\Theta$ = 3.6 (sec·g/cc), and the acrylonitrile (written as "AN" in Table 2) selectivity was 85.4% and the acrylonitrile yield was 84.7% at a conversion ratio of propylene of 99.2% to show that these values are almost in a range of variation in the analysis, and thus the results showing that the reaction performance of the catalyst was stable were obtained. The reaction results are shown in Table 2.

[Example 7]

A catalyst represented by the atomic ratios of respective elements and the mass of silica described in Examples 7, 8 in Table 1 was prepared in the manner as described below.

In a stirring and mixing vessel, 1333 g of aqueous silica sol containing 30% by mass of $SiO_2$ having an average particle diameter of silica primary particles of 12 nm was charged and stirred. It is to be noted that the primary particle diameters and the particle diameter distribution of the silica sol starting material used were determined by the BET method and the electron microscopic method to find that the average particle diameter of the primary particles was 12.1 nm, the standard deviation was 0.4 nm, and the ratio of the standard deviation to the average particle diameter was 3%.

Next, 200 g of an 8 wt% oxalic acid aqueous solution was added to the mixed liquid of the silica sol which was under stirring. Further, a liquid obtained by dissolving 465.4 g of ammonium paramolybdate [$(NH_4)_6Mo_7O_{24}·4H_2O$] in 830 g of water was added in the stirring and mixing vessel to obtain a mixed liquid. Subsequently, a liquid obtained by dissolving 75.26 g of bismuth nitrate [$Bi(NO_3)_3·5H_2O$] , 80.68 g of iron nitrate [$Fe(NO_3)_3·9H_2O$], 97.09 g of nickel nitrate [$Ni(NO_3)_2·6H_2O$], 34.23 g of magnesium nitrate [$Mg(NO_3)_2·6H_2O$], 453.5 g of cobalt nitrate [$Co(NO_3)_2·6H_2O$], 66.36 g of cerium nitrate [$Ce(NO_3)_3·6H_2O$], and 4.508 g of rubidium nitrate [$RbNO_3$] in 393 g of nitric acid the concentration of which is 16.6% by mass was added in the stirring and mixing vessel to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 590°C for 2 hours under an air atmosphere (third step) to finally obtain 827 g of a catalyst.

The results that the obtained catalyst had a solid sphere shape, an average particle diameter of 55 μm, and an apparent specific gravity of 1.01 g/cc were obtained. Next, ammoxidation reaction of propylene was performed using 50 g of the catalyst at a contact time of $\Theta$ = 3.8 (sec·g/cc). The conversion ratio of propylene was 99.2%, the acrylonitrile (written as "AN" in Table 2) selectivity was 84.9%, and the acrylonitrile yield was 84.2% at 24 hours after the start of the reaction.

In addition, the attrition strength was measured for 50 g of the catalyst in accordance with the ACC method to find that the attrition loss (%) was 0.7%. Further, when the sample after the attrition strength test was analyzed by X-ray diffraction, it was ascertained that the crystal phase of nickel molybdate was the β type crystal phase, and the α type crystal phase was hardly present.

The catalyst composition (metal composition, the amount of silica ($SiO_2$) carrier, values of $\alpha$, $\beta$, $\gamma$, $\delta$, $\varepsilon$) is shown in Table 1, and the reaction results and the results of physical property measurement are shown in Table 2.

[Example 8]

The reaction performance after 300 Hr was checked by continuing the ammoxidation reaction of propylene in Example 7 while finely adjusting the contact time so that the conversion ratio of propylene was maintained as it was to find that the contact time was almost the same at $\Theta$ = 3.9 (sec·g/cc), and the acrylonitrile (written as "AN" in Table 2) selectivity was 84.9% and the acrylonitrile yield was 84.2% at a conversion ratio of propylene of 99.2% to show that these values are almost in a range of variation in the analysis, and thus the results showing that the reaction performance of the catalyst was stable were obtained. The reaction results are shown in Table 2.

[Example 9]

A catalyst represented by the atomic ratios of respective elements and the mass of silica described in Examples 9, 10 in Table 1 was prepared in the manner as described below.

In a stirring and mixing vessel, 1333 g of aqueous silica sol containing 30% by mass of $SiO_2$ having an average

particle diameter of silica primary particles of 12 nm was charged and stirred. It is to be noted that the primary particle diameters and the particle diameter distribution of the silica sol starting material used were determined by the BET method and the electron microscopic method to find that the average particle diameter of the primary particles was 11.7 nm, the standard deviation was 0.4 nm, and the ratio of the standard deviation to the average particle diameter was 3%.

Next, a liquid obtained by dissolving 497.1 g of ammonium paramolybdate [$(NH_4)_6Mo_7O_{24}\cdot4H_2O$] in 887 g of water was added in the stirring and mixing vessel to obtain a mixed liquid. Subsequently, a liquid obtained by adding 375 g of an 8 wt% oxalic acid aqueous solution to a liquid obtained by dissolving 57.43 g of bismuth nitrate [$Bi(NO_3)_3\cdot5H_2O$], 191.5 g of iron nitrate [$Fe(NO_3)_3\cdot9H_2O$], 55.31 g of nickel nitrate [$Ni(NO_3)_2\cdot6H_2O$], 91.40 g of magnesium nitrate [$Mg(NO_3)_2\cdot6H_2O$], 346.05 g of cobalt nitrate [$Co(NO_3)_2\cdot6H_2O$], 40.51 g of cerium nitrate [$Ce(NO_3)_3\cdot6H_2O$], and 4.128 g of rubidium nitrate [$RbNO_3$] in 400 g of nitric acid the concentration of which is 16.6% by mass was put into the stirring and mixing vessel to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 600°C for 2 hours under an air atmosphere (third step) to finally obtain 829 g of a catalyst.

The results that the obtained catalyst had a solid sphere shape, an average particle diameter of 52 μm, and an apparent specific gravity of 1.01 g/cc were obtained. Next, ammoxidation reaction of propylene was performed using 50 g of the catalyst at a contact time of Θ = 3.6 (sec·g/cc). The conversion ratio of propylene was 99.2%, the acrylonitrile (written as "AN" in Table 2) selectivity was 85.2%, and the acrylonitrile yield was 84.5% at 24 hours after the start of the reaction.

In addition, the attrition strength was measured for 50 g of the catalyst in accordance with the ACC method to find that the attrition loss (%) was 0.8%.

Further, when the sample after the attrition strength test was analyzed by X-ray diffraction, it was ascertained that the crystal phase of nickel molybdate was the β type crystal phase, and the α type crystal phase was hardly present.

The catalyst composition (metal composition, the amount of silica ($SiO_2$) carrier, values of α, β, γ, δ, ε) is shown in Table 1, and the reaction results and the results of physical property measurement are shown in Table 2.

[Example 10]

The reaction performance after 300 Hr was checked by continuing the ammoxidation reaction of propylene in Example 9 while finely adjusting the contact time so that the conversion ratio of propylene was maintained as it was to find that the contact time was almost the same at Θ = 3.8 (sec·g/cc), and the acrylonitrile (written as "AN" in Table 2) selectivity was 85.3% and the acrylonitrile yield was 84.5% at a conversion ratio of propylene of 99.1% to show that these values are almost in a range of variation in the analysis, and thus the results showing that the reaction performance of the catalyst was stable were obtained. The reaction results are shown in Table 2.

[Example 11]

A catalyst represented by the atomic ratios of respective elements and the mass of silica described in Examples 11, 12 in Table 1 was prepared in the manner as described below.

In a stirring and mixing vessel, 1333 g of aqueous silica sol containing 30% by mass of $SiO_2$ having an average particle diameter of silica primary particles of 12 nm was charged and stirred. It is to be noted that the primary particle diameters and the particle diameter distribution of the silica sol starting material used were determined by the BET method and the electron microscopic method to find that the average particle diameter of the primary particles was 12.2 nm, the standard deviation was 0.6 nm, and the ratio of the standard deviation to the average particle diameter was 5%.

Next, 320 g of an 8 wt% oxalic acid aqueous solution was added to the mixed liquid of the silica sol which was under stirring. Further, a liquid obtained by dissolving 474.9 g of ammonium paramolybdate [$(NH_4)_6Mo_7O_{24}\cdot4H_2O$] in 847 g of water was added in the stirring and mixing vessel to obtain a mixed liquid. Subsequently, a liquid obtained by dissolving 65.83 g of bismuth nitrate [$Bi(NO_3)_3\cdot5H_2O$] , 73.18 g of iron nitrate [$Fe(NO_3)_3\cdot9H_2O$], 79.26 g of nickel nitrate [$Ni(NO_3)_2\cdot6H_2O$], 58.21 g of magnesium nitrate [$Mg(NO_3)_2\cdot6H_2O$], 396.7 g of cobalt nitrate [$Co(NO_3)_2\cdot6H_2O$], 87.07 g of cerium nitrate [$Ce(NO_3)_3\cdot6H_2O$], 11.31 g of potassium nitrate [$KNO_3$], and 4.382 g of cesium nitrate [$CsNO_3$] in 390 g of nitric acid the concentration of which is 16.6% by mass was added in the stirring and mixing vessel to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 600°C for 2 hours under an air atmosphere (third step) to finally obtain 818 g of a catalyst.

The results that the obtained catalyst had a solid sphere shape, an average particle diameter of 53 $\mu$m, and an apparent specific gravity of 1.04 g/cc were obtained. Next, ammoxidation reaction of propylene was performed using 50 g of the catalyst at a contact time of $\Theta$ = 4.0 (sec·g/cc). The conversion ratio of propylene was 99.3%, the acrylonitrile (written as "AN" in Table 2) selectivity was 85.5%, and the acrylonitrile yield was 84.9% at 24 hours after the start of the reaction.

In addition, the attrition strength was measured for 50 g of the catalyst in accordance with the ACC method to find that the attrition loss (%) was 0.6%.

Further, when the sample after the attrition strength test was analyzed by X-ray diffraction, it was ascertained that the crystal phase of nickel molybdate was the $\beta$ type crystal phase, and the $\alpha$ type crystal phase was hardly present.

The catalyst composition (metal composition, the amount of silica ($SiO_2$) carrier, values of $\alpha$, $\beta$, $\gamma$, $\delta$, $\varepsilon$) is shown in Table 1, and the reaction results and the results of physical property measurement are shown in Table 2.

[Example 12]

The reaction performance after 300 Hr was checked by continuing the ammoxidation reaction of propylene in Example 11 while finely adjusting the contact time so that the conversion ratio of propylene was maintained as it was to find that the contact time was almost the same at $\Theta$ = 4.1 (sec·g/cc), and the acrylonitrile (written as "AN" in Table 2) selectivity was 85.4% and the acrylonitrile yield was 84.8% at a conversion ratio of propylene of 99.3%, and thus the high reactivity was maintained. The reaction results are shown in Table 2.

[Example 13]

A catalyst represented by the atomic ratios of respective elements and the mass of silica described in Examples 13, 14 in Table 1 was prepared in the manner as described below.

In a stirring and mixing vessel, 1333 g of aqueous silica sol containing 30% by mass of $SiO_2$ having an average particle diameter of silica primary particles of 12 nm was charged and stirred. It is to be noted that the primary particle diameters and the particle diameter distribution of the silica sol starting material used were determined by the BET method and the electron microscopic method to find that the average particle diameter of the primary particles was 11.9 nm, the standard deviation was 0.4 nm, and the ratio of the standard deviation to the average particle diameter was 3%.

Next, 430 g of an 8 wt% oxalic acid aqueous solution was added to the mixed liquid of the silica sol which was under stirring. Further, a liquid obtained by dissolving 486.7 g of ammonium paramolybdate [$(NH_4)_6Mo_7O_{24} \cdot 4H_2O$] in 870 g of water was added in the stirring and mixing vessel to obtain a mixed liquid. Subsequently, a liquid obtained by adding 250 g of an 8 wt% tartaric acid aqueous solution to a liquid obtained by dissolving 39.36 g of bismuth nitrate [$Bi(NO_3)_3 \cdot 5H_2O$], 93.75 g of iron nitrate [$Fe(NO_3)_3 \cdot 9H_2O$], 81.23 g of nickel nitrate [$Ni(NO_3)_2 \cdot 6H_2O$], 59.66 g of magnesium nitrate [$Mg(NO_3)_2 \cdot 6H_2O$], 372.7 g of cobalt nitrate [$CO(NO_3)_2 \cdot 6H_2O$], 89.24 g of cerium nitrate [$Ce(NO_3)_3 \cdot 6H_2O$], and 4.042 g of rubidium nitrate [$RbNO_3$] in 395 g of nitric acid the concentration of which is 16.6% by mass was put into the stirring and mixing vessel to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 600°C for 2 hours under an air atmosphere (third step) to finally obtain 825 g of a catalyst.

The results that the obtained catalyst had a solid sphere shape, an average particle diameter of 52 $\mu$m, and an apparent specific gravity of 1.02 g/cc were obtained. Next, ammoxidation reaction of propylene was performed using 50 g of the catalyst at a contact time of $\Theta$ = 3.7 (sec·g/cc). The conversion ratio of propylene was 99.3%, the acrylonitrile (written as "AN" in Table 2) selectivity was 85.2%, and the acrylonitrile yield was 84.6% at 24 hours after the start of the reaction.

In addition, the attrition strength was measured for 50 g of the catalyst in accordance with the ACC method to find that the attrition loss (%) showed a low value, as low as 0.4%.

Further, when the sample after the attrition strength test was analyzed by X-ray diffraction, it was ascertained that the crystal phase of nickel molybdate was the $\beta$ type crystal phase, and the $\alpha$ type crystal phase was hardly present.

The catalyst composition (metal composition, the amount of silica ($SiO_2$) carrier, values of $\alpha$, $\beta$, $\gamma$, $\delta$, $\varepsilon$) is shown in Table 1, and the reaction results and the results of physical property measurement are shown in Table 2.

[Example 14]

The reaction performance after 300 Hr was checked by continuing the ammoxidation reaction of propylene in Example 13 while finely adjusting the contact time so that the conversion ratio of propylene was maintained as it was to find that the contact time was almost the same at $\Theta$ = 3.8 (sec·g/cc), and the acrylonitrile (written as "AN" in Table 2) selectivity was 85.1% and the acrylonitrile yield was 84.4% at a conversion ratio of propylene of 99.2%, and thus the high reactivity was maintained. The reaction results are shown in Table 2.

[Example 15]

A catalyst represented by the atomic ratios of respective elements and the mass of silica described in Examples 15, 16 in Table 1 was prepared in the manner as described below.

A mixed liquid of two kinds of silica sol was obtained by mixing 1067 g of aqueous silica sol containing 30% by mass of $SiO_2$ having an average particle diameter of silica primary particles of 12 nm and 200 g of aqueous silica sol containing 40% by mass of $SiO_2$ having an average particle diameter of silica primary particles of 41 nm. It is to be noted that the primary particle diameters and the particle diameter distribution of the starting material obtained by mixing two kinds of silica sol were determined by the BET method and the electron microscopic method to find that the average particle diameter of the primary particles was 13.9 nm, the standard deviation was 2.9 nm, and the ratio of the standard deviation to the average particle diameter was 21%.

Next, 250 g of an 8 wt% oxalic acid aqueous solution was added to the mixed liquid of the silica sol which was under stirring. Further, a liquid obtained by dissolving 496.4 g of ammonium paramolybdate [$(NH_4)_6Mo_7O_{24} \cdot 4H_2O$] in 1000 g of water was added to the first mixed liquid to obtain a second mixed liquid. Subsequently, a liquid obtained by dissolving 32.08 g of bismuth nitrate [$Bi(NO_3)_3 \cdot 5H_2O$] , 198 g of iron nitrate [$Fe(NO_3)_3 \cdot 9H_2O$], 68.84 g of nickel nitrate [$Ni(NO_3)_2 \cdot 6H_2O$], 24.34 g of magnesium nitrate [$Mg(NO_3)_2 \cdot 6H_2O$], 359.4 g of cobalt nitrate [$Co(NO_3)_2 \cdot 6H_2O$], 76.01 g of cerium nitrate [$Ce(NO_3)_3 \cdot 6H_2O$], and 5.497 g of rubidium nitrate [$RbNO_3$] in 410 g of nitric acid the concentration of which is 16.6% by mass was added to the second mixed liquid to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 585°C for 2 hours under an air atmosphere (third step) to finally obtain 806 g of a catalyst.

The results that the obtained catalyst had a solid sphere shape, an average particle diameter of 51 $\mu$m, and an apparent specific gravity of 1.01 g/cc were obtained. Next, ammoxidation reaction of propylene was performed using 50 g of the catalyst at a contact time of $\Theta$ = 4 (sec·g/cc). The conversion ratio of propylene was 99.2%, the acrylonitrile (written as "AN" in Table 2) selectivity was 85.1%, and the acrylonitrile yield was 84.4% at 24 hours after the start of the reaction.

In addition, the attrition strength was measured for 50 g of the catalyst in accordance with the ACC method to find that the attrition loss (%) showed a low value, as low as 0.4%.

Further, when the sample after the attrition strength test was analyzed by X-ray diffraction, it was ascertained that the crystal phase of nickel molybdate was the $\beta$ type crystal phase, and the $\alpha$ type crystal phase was hardly present.

The catalyst composition (metal composition, the amount of silica ($SiO_2$) carrier, values of $\alpha$, $\beta$, $\gamma$, $\delta$, $\varepsilon$) is shown in Table 1, and the reaction results and the results of physical property measurement are shown in Table 2.

[Example 16]

The reaction performance after 300 Hr was checked by continuing the ammoxidation reaction of propylene in Example 15 while finely adjusting the contact time so that the conversion ratio of propylene was maintained as it was to find that the contact time was almost the same at $\Theta$ = 4 (sec·g/cc), and the acrylonitrile (written as "AN" in Table 2) selectivity was 85.3% and the acrylonitrile yield was 84.6% at a conversion ratio of propylene of 99.2%, and thus the high reactivity was maintained. The reaction results are shown in Table 2.

[Example 17]

A catalyst represented by the atomic ratios of respective elements and the mass of silica described in Examples 17, 18 in Table 1 was prepared in the manner as described below.

A mixed liquid of two kinds of silica sol was obtained by mixing 863.3 g of aqueous silica sol containing 30%

by mass of $SiO_2$ having an average particle diameter of silica primary particles of 12 nm and 277.5 g of aqueous silica sol containing 40% by mass of $SiO_2$ having an average particle diameter of silica primary particles of 41 nm. It is to be noted that the primary particle diameters and the particle diameter distribution of the starting material obtained by mixing two kinds of silica sol were determined by the BET method and the electron microscopic method to find that the average particle diameter of the primary particles was 15.6 nm, the standard deviation was 4.4 nm, and the ratio of the standard deviation to the average particle diameter was 28%.

Next, 250 g of an 8 wt% oxalic acid aqueous solution was added to the mixed liquid of the silica sol which was under stirring. Further, a liquid obtained by dissolving 527.1 g of ammonium paramolybdate [$(NH_4)_6Mo_7O_{24}\cdot4H_2O$] in 1100 g of water was added to the first mixed liquid to obtain a second mixed liquid. Subsequently, a liquid obtained by dissolving 48.67 g of bismuth nitrate [$Bi(NO_3)_3\cdot5H_2O$], 230.3 g of iron nitrate [$Fe(NO_3)_3\cdot9H_2O$], 131.6 g of nickel nitrate [$Ni(NO_3)_2\cdot6H_2O$], 19.38 g of magnesium nitrate [$Mg(NO_3)_2\cdot6H_2O$], 256.9 g of cobalt nitrate [$Co(NO_3)_2\cdot6H_2O$], 80.7 g of cerium nitrate [$Ce(NO_3)_3\cdot6H_2O$], and 5.472 g of rubidium nitrate [$RbNO_3$] in 421 g of nitric acid the concentration of which is 16.6% by mass was added to the second mixed liquid to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 590°C for 2 hours under an air atmosphere (third step) to finally obtain 806 g of a catalyst.

The results that the obtained catalyst had a solid sphere shape, an average particle diameter of 54 $\mu$m, and an apparent specific gravity of 1.02 g/cc were obtained. Next, ammoxidation reaction of propylene was performed using 50 g of the catalyst at a contact time of $\Theta$ = 3.9 (sec·g/cc). The conversion ratio of propylene was 99.3%, the acrylonitrile (written as "AN" in Table 2) selectivity was 84.7%, and the acrylonitrile yield was 84.1% at 24 hours after the start of the reaction.

In addition, the attrition strength was measured for 50 g of the catalyst in accordance with the ACC method to find that the attrition loss (%) showed a low value, as low as 0.5%.

Further, when the sample after the attrition strength test was analyzed by X-ray diffraction, it was ascertained that the crystal phase of nickel molybdate was the β type crystal phase, and the α type crystal phase was hardly present.

The catalyst composition (metal composition, the amount of silica ($SiO_2$) carrier, values of α, β, γ, δ, ε) is shown in Table 1, and the reaction results and the results of physical property measurement are shown in Table 2.

[Example 18]

The reaction performance after 300 Hr was checked by continuing the ammoxidation reaction of propylene in Example 17 while finely adjusting the contact time so that the conversion ratio of propylene was maintained as it was to find that the contact time was almost the same at $\Theta$ = 4 (sec·g/cc), and the acrylonitrile (written as "AN" in Table 2) selectivity was 84.9% and the acrylonitrile yield was 84.2% at a conversion ratio of propylene of 99.2%, and thus the high reactivity was maintained. The reaction results are shown in Table 2.

[Comparative Example 1]

A catalyst represented by the atomic ratios of respective elements and the mass of silica described in Comparative Examples 1, 2 in Table 1 was prepared in the manner as described below.

In a stirring and mixing vessel, 1333 g of aqueous silica sol containing 30% by mass of $SiO_2$ having an average particle diameter of silica primary particles of 12 nm was charged and stirred. It is to be noted that the primary particle diameters and the particle diameter distribution of the silica sol starting material used were determined by the BET method and the electron microscopic method to find that the average particle diameter of the primary particles was 12 nm, the standard deviation was 0.4 nm, and the ratio of the standard deviation to the average particle diameter was 3%.

Next, 250 g of an 8 wt% oxalic acid aqueous solution was added in the stirring and mixing vessel charged with the silica sol which was under stirring. Next, a liquid obtained by dissolving 492.6 g of ammonium paramolybdate [$(NH_4)_6Mo_7O_{24}\cdot4H_2O$] in 880 g of water was added in the stirring and mixing vessel to obtain a mixed liquid. Subsequently, a liquid obtained by dissolving 45.53 g of bismuth nitrate [$Bi(NO_3)_3\cdot5H_2O$], 56.94 g of iron nitrate [$Fe(NO_3)_3\cdot9H_2O$], 397.4 g of nickel nitrate [$Ni(NO_3)_2\cdot6H_2O$], 233.2 g of cobalt nitrate [$Co(NO_3)_2\cdot6H_2O$], 45.53 g of cerium nitrate [$Ce(NO_3)_3\cdot6H_2O$], and 3.409 g of rubidium nitrate [$RbNO_3$] in 400 g of nitric acid the concentration of which is 16.6% by mass was added in the stirring and mixing vessel to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 570°C for 2 hours under an air atmosphere (third step) to finally obtain 824 g of a catalyst.

The results that the obtained catalyst had a solid sphere shape, an average particle diameter of 53 $\mu$m, and an apparent specific gravity of 1.02 g/cc were obtained. Next, ammoxidation reaction of propylene was performed using 50 g of the catalyst at a contact time of $\Theta$ = 4.3 (sec·g/cc). The conversion ratio of propylene was 99.0%, the acrylonitrile (written as "AN" in Table 2) selectivity was 83.8%, and the acrylonitrile yield was 83.0% at 24 hours after the start of the reaction.

In addition, the attrition strength was measured for 50 g of the catalyst in accordance with the ACC method to find that the attrition loss (%) showed a low value, as low as 0.3%.

Further, when the sample after the attrition strength test was analyzed by X-ray diffraction, it was ascertained that the crystal phase of nickel molybdate was the $\beta$ type crystal phase, and the $\alpha$ type crystal phase was hardly present.

The catalyst composition (metal composition, the amount of silica ($SiO_2$) carrier, values of $\alpha$, $\beta$, y, $\delta$, $\varepsilon$) is shown in Table 1, and the reaction results and the results of physical property measurement are shown in Table 2.

[Comparative Example 2]

The reaction performance after 300 Hr was checked by continuing the ammoxidation reaction of propylene in Comparative Example 1 while finely adjusting the contact time so that the conversion ratio of propylene was maintained as it was to find that the contact time was deteriorated to $\Theta$ = 4.8 (sec·g/cc), and the acrylonitrile (written as "AN" in Table 2) selectivity was 83.2 and the acrylonitrile yield was 82.5% at a conversion ratio of propylene of 99.2%, and thus it was observed that these values are slightly lowered. The reaction results are shown in Table 2.

[Comparative Example 3]

A catalyst represented by the atomic ratios of respective elements and the mass of silica described in Comparative Examples 3 in Table 1 was prepared in the manner as described below.

In a stirring and mixing vessel, 1333 g of aqueous silica sol containing 30% by mass of $SiO_2$ having an average particle diameter of silica primary particles of 12 nm was charged and stirred. It is to be noted that the primary particle diameters and the particle diameter distribution of the silica sol starting material used were determined by the BET method and the electron microscopic method to find that the average particle diameter of the primary particles was 12.1 nm, the standard deviation was 0.5 nm, and the ratio of the standard deviation to the average particle diameter was 4%.

Next, 200 g of a 10 wt% tartaric acid aqueous solution was added in the stirring and mixing vessel charged with the silica sol which was under stirring. Next, a liquid obtained by dissolving 479.0 g of ammonium paramolybdate $[(NH_4)_6Mo_7O_{24}\cdot4H_2O]$ in 860 g of water was added in the stirring and mixing vessel to obtain a mixed liquid. Subsequently, a liquid obtained by dissolving 60.87 g of bismuth nitrate $[Bi(NO_3)_3\cdot5H_2O]$, 184.5 g of iron nitrate $[Fe(NO_3)_3\cdot9H_2O]$, 466.4 g of nickel nitrate $[Ni(NO_3)_2\cdot6H_2O]$, 66.692 g of cobalt nitrate $[Co(NO_3)_2\cdot6H_2O]$, 29.28 g of cerium nitrate $[Ce(NO_3)3\cdot6H_2O]$, and 3.647 g of rubidium nitrate $[RbNO_3]$ in 400 g of nitric acid the concentration of which is 16.6% by mass was added in the stirring and mixing vessel to obtain an aqueous raw material mixture (starting material slurry) (first step). Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 600°C for 2 hours under an air atmosphere (third step) to finally obtain 827 g of a catalyst.

The results that the obtained catalyst had a solid sphere shape, an average particle diameter of 54 $\mu$m, and an apparent specific gravity of 1.02 g/cc were obtained. Next, ammoxidation reaction of propylene was performed using 50 g of the catalyst at a contact time of $\Theta$ = 4.1 (sec·g/cc). The conversion ratio of propylene was 99.2%, the acrylonitrile (written as "AN" in Table 2) selectivity was 83.1%, and the acrylonitrile yield was 82.4% at 24 hours after the start of the reaction.

In addition, the attrition strength was measured for 50 g of the catalyst in accordance with the ACC method to find that the attrition loss (%) showed a low value, as low as 0.4%.

Further, when the sample after the attrition strength test was analyzed by X-ray diffraction, the $\alpha$ type crystal phase was observed in addition to the $\beta$ crystal phase in the crystal phases of nickel molybdate.

The catalyst composition (metal composition, the amount of silica ($SiO_2$) carrier, values of $\alpha$, $\beta$, $\gamma$, $\delta$) is shown in Table 1, and the reaction results and the results of physical property measurement are shown in Table 2.

[Comparative Example 4]

A catalyst represented by the atomic ratios of respective elements and the mass of silica described in Comparative Examples 4 in Table 1 was prepared in the manner as described below.

In a stirring and mixing vessel, 1333.3 g of aqueous silica sol containing 30% by mass of $SiO_2$ having an average particle diameter of silica primary particles of 12 nm was charged and stirred. It is to be noted that the primary particle diameters and the particle diameter distribution of the silica sol starting material used were determined by the BET method and the electron microscopic method to find that the average particle diameter of the primary particles was 12.3 nm, the standard deviation was 0.6 nm, and the ratio of the standard deviation to the average particle diameter was 5%.

Next, 200 g of an 8 wt% oxalic acid aqueous solution was added, and the resultant mixture was stirred and mixed. Next, a liquid obtained by dissolving 470.9 g of ammonium paramolybdate [$(NH_4)_6Mo_7O_{24}\cdot4H_2O$] in 840 g of water was blended, and the resultant mixture was stirred. Subsequently, a liquid obtained by dissolving 97.93 g of bismuth nitrate [$Bi(NO_3)_3\cdot5H_2O$], 145.1 g of iron nitrate [$Fe(NO_3)_3\cdot9H_2O$], 196. 5 g of nickel nitrate [$Ni(NO_3)_2\cdot6H_2O$], 46.18 g of magnesium nitrate [$Mg(NO_3)_2\cdot6H_2O$], 249.15 g of cobalt nitrate [$Co(NO_3)_2\cdot6H_2O$], 57.57 g of cerium nitrate [$Ce(NO_3)_3\cdot6H_2O$], and 4.889 g of rubidium nitrate [$RbNO_3$] in 400 g of nitric acid the concentration of which is 16.6% by mass was added in the stirring and mixing vessel to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 580°C for 2 hours under an air atmosphere (third step) to finally obtain 828 g of a catalyst.

The results that the obtained catalyst had a solid sphere shape, an average particle diameter of 55 μm, and an apparent specific gravity of 1.01 g/cc were obtained. Next, ammoxidation reaction of propylene was performed using 50 g of the catalyst at a contact time of $\Theta$ = 3.9 (sec·g/cc). The conversion ratio of propylene was 99.2%, the acrylonitrile (written as "AN" in Table 2) selectivity was 83.6%, and the acrylonitrile yield was 82.9% at 24 hours after the start of the reaction.

In addition, the attrition strength was measured for 50 g of the catalyst in accordance with the ACC method to find that the attrition loss (%) was 0.9%.

Further, when the sample after the attrition strength test was analyzed by X-ray diffraction, it was ascertained that the crystal phase of nickel molybdate was the β type crystal phase, and the α type crystal phase was hardly present.

The catalyst composition (metal composition, the amount of silica ($SiO_2$) carrier, values of α, β, γ, δ) is shown in Table 1, and the reaction results and the results of physical property measurement are shown in Table 2.

[Comparative Example 5]

A catalyst represented by the atomic ratios of respective elements and the mass of silica described in Comparative Examples 5 in Table 1 was prepared in the manner as described below.

A mixed liquid of two kinds of silica sol was obtained by mixing 666.7 g of aqueous silica sol containing 30% by mass of $SiO_2$ having an average particle diameter of silica primary particles of 12 nm and 500 g of aqueous silica sol containing 40% by mass of $SiO_2$ having an average particle diameter of silica primary particles of 41 nm. It is to be noted that the primary particle diameters and the particle diameter distribution of the starting material obtained by mixing two kinds of silica sol were determined by the BET method and the electron microscopic method to find that the average particle diameter of the primary particles was 19 nm, the standard deviation was 7.9 nm, and the ratio of the standard deviation to the average particle diameter was 42%.

Next, 250 g of an 8 wt% oxalic acid aqueous solution was added to the mixed liquid of the silica sol which was under stirring to obtain a first mixed liquid. Next, a liquid obtained by dissolving 491.6 g of ammonium paramolybdate [$(NH_4)_6Mo_7O_{24}\cdot4H_2O$] in 877 g of water was added in the stirring and mixing vessel to obtain a mixed liquid. Subsequently, a liquid obtained by dissolving 45.44 g of bismuth nitrate [$Bi(NO_3)_3\cdot5H_2O$], 189.4 g of iron nitrate [$Fe(NO_3)_3\cdot9H_2O$], 20.51 g of nickel nitrate [$Ni(NO_3)_2\cdot6H_2O$], 48.21 g of magnesium nitrate [$Mg(NO_3)_2\cdot6H_2O$], 376.44 g of cobalt nitrate [$Co(NO_3)_2\cdot6H_2O$], 90.14 g of cerium nitrate [$Ce(NO_3)_3\cdot6H_2O$], and 3.402 g of rubidium nitrate [$RbNO_3$] in 400 g of nitric acid the concentration of which is 16.6% by mass was added in the stirring and mixing vessel to obtain an aqueous raw material mixture (starting material slurry) (first step). Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried catalyst precursor was preliminarily calcined using an electric furnace at 320°C for 2 hours under an air atmosphere and was then finally calcined at 600°C for 2 hours under an air atmosphere (third step) to finally obtain 830 g of a catalyst.

The results that the obtained catalyst had a solid sphere shape, an average particle diameter of 53 $\mu$m, and an apparent specific gravity of 0.98 g/cc were obtained. Next, ammoxidation reaction of propylene was performed using 50 g of the catalyst at a contact time of $\Theta$ = 4.2 (sec·g/cc). The conversion ratio of propylene was 99.2%, the acrylonitrile (written as "AN" in Table 2) selectivity was 82.9%, and the acrylonitrile yield was 82.2% at 24 hours after the start of the reaction.

In addition, the attrition strength was measured for 50 g of the catalyst in accordance with the ACC method to find that the attrition loss (%) was 1.7%.

Further, when the sample after the attrition strength test was analyzed by X-ray diffraction, it was ascertained that the crystal phase of nickel molybdate was the $\beta$ type crystal phase, and the $\alpha$ type crystal phase was hardly present.

The catalyst composition (metal composition, the amount of silica (SiO$_2$) carrier, values of $\alpha$, $\beta$, $\gamma$, $\delta$) is shown in Table 1, and the reaction results and the results of physical property measurement are shown in Table 2.

[Comparative Example 6]

A catalyst represented by the atomic ratios of respective elements and the mass of silica described in Comparative Examples 6 in Table 1 was prepared in the manner as described below.

In a stirring and mixing vessel, 1333 g of aqueous silica sol containing 30% by mass of SiO$_2$ having an average particle diameter of silica primary particles of 12 nm was charged and stirred. It is to be noted that the primary particle diameters and the particle diameter distribution of the silica sol starting material used were determined by the BET method and the electron microscopic method to find that the average particle diameter of the primary particles was 12.2 nm, the standard deviation was 0.6 nm, and the ratio of the standard deviation to the average particle diameter was 5%.

Next, a liquid obtained by dissolving 494.9 g of ammonium paramolybdate [(NH$_4$)$_6$Mo$_7$O$_{24}$·4H$_2$O] in 880 g of water was added in the stirring and mixing vessel to obtain a mixed liquid. Subsequently, a liquid obtained by dissolving 17.15 g of bismuth nitrate [Bi(NO$_3$)$_3$·5H$_2$O], 152.5 g of iron nitrate [Fe(NO$_3$)$_3$·9H$_2$O], 309.7 g of nickel nitrate [Ni(NO$_3$)$_2$·6H$_2$O], 60.66 g of magnesium nitrate [Mg(NO$_3$)$_2$·6H$_2$O], 192.9 g of cobalt nitrate [Co(NO$_3$)$_2$·6H$_2$O], 60.49 g of cerium nitrate [Ce(NO$_3$)$_3$·6H$_2$O], and 4.452 g of rubidium nitrate [RbNO$_3$] in 400 g of nitric acid the concentration of which is 16.6% by mass was added in the stirring and mixing vessel, and further, 200 g of 10 % tartaric acid was added to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried catalyst precursor was preliminarily calcined using an electric furnace at 320°C for 2 hours under an air atmosphere and was then finally calcined at 620°C for 2 hours under an air atmosphere (third step) to finally obtain 834 g of a catalyst.

The results that the obtained catalyst had a solid sphere shape, an average particle diameter of 54 $\mu$m, and an apparent specific gravity of 1.00 g/cc were obtained. Next, ammoxidation reaction of propylene was performed using 50 g of the catalyst at a contact time of $\Theta$ = 4.1 (sec·g/cc). The conversion ratio of propylene was 99.1%, the acrylonitrile (written as "AN" in Table 2) selectivity was 83.6%, and the acrylonitrile yield was 82.8% at 24 hours after the start of the reaction.

In addition, the attrition strength was measured for 50 g of the catalyst in accordance with the ACC method to find that the attrition loss (%) was 1.1%.

Further, when the sample after the attrition strength test was analyzed by X-ray diffraction, it was ascertained that the crystal phase of nickel molybdate was the $\beta$ type crystal phase, and the $\alpha$ type crystal phase was hardly present.

The catalyst composition (metal composition, the amount of silica (SiO$_2$) carrier, values of $\alpha$, $\beta$, $\gamma$, $\delta$) is shown in Table 1, and the reaction results and the results of physical property measurement are shown in Table 2.

[Comparative Example 7]

A catalyst represented by the atomic ratios of respective elements and the mass of silica described in Comparative Examples 7 in Table 1 was prepared in the manner as described below.

A mixed liquid of two kinds of silica sol was obtained by mixing 666.7 g of aqueous silica sol containing 30% by mass of SiO$_2$ having an average particle diameter of silica primary particles of 12 nm and 500 g of aqueous silica sol containing 40% by mass of SiO$_2$ having an average particle diameter of silica primary particles of 41 nm. It is to be noted that the primary particle diameters and the particle diameter distribution of the starting material obtained by mixing two kinds of silica sol were determined by the BET method and the electron microscopic method to find that the average particle diameter of the primary particles was 18.5 nm, the standard deviation was 7.3 nm, and the ratio of the standard deviation to the average particle diameter was 40%.

Next, a liquid obtained by dissolving 489.0 g of ammonium paramolybdate [$(NH_4)_6Mo_7O_{24}\cdot4H_2O$] in 870 g of water was added in the stirring and mixing vessel to obtain a mixed liquid. Subsequently, a liquid obtained by adding 45.20 g of bismuth nitrate [$Bi(NO_3)_3\cdot5H_2O$], 329.7 g of iron nitrate [$Fe(NO_3)_3\cdot9H_2O$], 197.3 g of nickel nitrate [$Ni(NO_3)_2\cdot6H_2O$], 137.9 g of magnesium nitrate [$Mg(NO_3)_2\cdot6H_2O$], 136.2 g of cobalt nitrate [$Co(NO_3)_2\cdot6H_2O$], 29.89 g of cerium nitrate [$Ce(NO_3)_3\cdot6H_2O$], 3.384 g of rubidium nitrate [$RbNO_3$], and 200 g of an 8 wt% oxalic acid aqueous solution to 410 g of nitric acid the concentration of which is 16.6% by mass and mixing the resultant mixture was added in the stirring and mixing vessel to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 600°C for 2 hours under an air atmosphere (third step) to finally obtain 822 g of a catalyst.

The results that the obtained catalyst had a solid sphere shape, an average particle diameter of 53 $\mu$m, and an apparent specific gravity of 0.99 g/cc were obtained. Next, ammoxidation reaction of propylene was performed using 50 g of the catalyst at a contact time of $\Theta$ = 4.1 (sec·g/cc). The conversion ratio of propylene was 99.1%, the acrylonitrile (written as "AN" in Table 2) selectivity was 82.9%, and the acrylonitrile yield was 82.2% at 24 hours after the start of the reaction.

In addition, the attrition strength was measured for 50 g of the catalyst in accordance with the ACC method to find that the attrition loss (%) was 1.5%.

Further, when the sample after the attrition strength test was analyzed by X-ray diffraction, it was ascertained that the crystal phase of nickel molybdate was the $\beta$ type crystal phase, and the $\alpha$ type crystal phase was hardly present.

The catalyst composition (metal composition, the amount of silica ($SiO_2$) carrier, values of $\alpha$, $\beta$, $\gamma$, $\delta$, $\varepsilon$) is shown in Table 1, and the reaction results and the results of physical property measurement are shown in Table 2.

[Comparative Example 8]

A catalyst represented by the atomic ratios of respective elements and the mass of silica described in Comparative Examples 8 in Table 1 was prepared in the manner as described below.

In a stirring and mixing vessel, 1333 g of aqueous silica sol containing 30% by mass of $SiO_2$ having an average particle diameter of silica primary particles of 12 nm was charged and stirred. It is to be noted that the primary particle diameters and the particle diameter distribution of the silica sol starting material used were determined by the BET method and the electron microscopic method to find that the average particle diameter of the primary particles was 12 nm, the standard deviation was 0.5 nm, and the ratio of the standard deviation to the average particle diameter was 4%.

Next, 200 g of an 8 wt% oxalic acid aqueous solution was added in the stirring and mixing vessel to make a mixed liquid of silica and oxalic acid. Next, a liquid obtained by dissolving 482.5 g of ammonium paramolybdate [$(NH_4)_6Mo_7O_{24}\cdot4H_2O$] in 860 g of water was added in the stirring and mixing vessel to obtain a mixed liquid. Subsequently, a liquid obtained by dissolving 27.87 g of bismuth nitrate [$Bi(NO_3)_3\cdot5H_2O$], 130.1 g of iron nitrate [$Fe(NO_3)_3\cdot9H_2O$], 268.5 g of nickel nitrate [$Ni(NO_3)_2\cdot6H_2O$], 136.0 g of magnesium nitrate [$Mg(NO_3)_2\cdot6H_2O$], 201.5 g of cobalt nitrate [$Co(NO_3)_2\cdot6H_2O$], 68.81 g of cerium nitrate [$Ce(NO_3)_3\cdot6H_2O$], and 3.339 g of rubidium nitrate [$RbNO_3$] in 400 g of nitric acid the concentration of which is 16.6% by mass was added in the stirring and mixing vessel to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 630°C for 2 hours under an air atmosphere (third step) to finally obtain 830 g of a catalyst.

The results that the obtained catalyst had a solid sphere shape, an average particle diameter of 51 $\mu$m, and an apparent specific gravity of 0.98 g/cc were obtained. Next, ammoxidation reaction of propylene was performed using 50 g of the catalyst at a contact time of $\Theta$ = 4.0 (sec·g/cc). The conversion ratio of propylene was 99.1%, the acrylonitrile (written as "AN" in Table 2) selectivity was 82.8%, and the acrylonitrile yield was 82.1% at 24 hours after the start of the reaction.

In addition, the attrition strength was measured for 50 g of the catalyst in accordance with the ACC method to find that the attrition loss (%) showed a low value, as low as 0.3%.

Further, when the sample after the attrition strength test was analyzed by X-ray diffraction, it was ascertained that the crystal phase of nickel molybdate was the $\beta$ type crystal phase, and the $\alpha$ type crystal phase was hardly present.

The catalyst composition (metal composition, the amount of silica ($SiO_2$) carrier, values of $\alpha$, $\beta$, $\gamma$, $\delta$) is shown in Table 1, and the reaction results and the results of physical property measurement are shown in Table 2.

[Comparative Example 9]

The reaction performance after 300 Hr was checked by continuing the ammoxidation reaction of propylene in Comparative Example 8 while finely adjusting the contact time so that the conversion ratio of propylene was maintained as it was to find that the contact time was deteriorated to $\Theta$ = 4.7 (sec·g/cc), and the acrylonitrile (written as "AN" in Table 2) selectivity was 82.5% and the acrylonitrile yield was 81.7% at a conversion ratio of propylene of 99.2%, and thus it was observed that these values were lowered. The reaction results are shown in Table 2.

[Comparative Example 10]

A catalyst represented by the atomic ratios of respective elements and the mass of silica described in Comparative Examples 8 in Table 1 was prepared in the manner as described below.

In a stirring and mixing vessel, 1667 g of aqueous silica sol containing 30% by mass of $SiO_2$ having an average particle diameter of silica primary particles of 12 nm was charged and stirred. It is to be noted that the primary particle diameters and the particle diameter distribution of the silica sol starting material used were determined by the BET method and the electron microscopic method to find that the average particle diameter of the primary particles was 11.9 nm, the standard deviation was 0.4 nm, and the ratio of the standard deviation to the average particle diameter was 3%.

Next, 300 g of an 8 wt% oxalic acid aqueous solution was added in the stirring and mixing vessel charged with the silica sol which was under stirring. Next, a liquid obtained by dissolving 404.2 g of ammonium paramolybdate $[(NH_4)_6Mo_7O_{24}\cdot4H_2O]$ in 720 g of water was added in the stirring and mixing vessel to obtain a mixed liquid. Subsequently, a liquid obtained by dissolving 32.69 g of bismuth nitrate $[Bi(NO_3))3\cdot5H_2O]$, 124.6 g of iron nitrate $[Fe(NO_3)_3\cdot9H_2O]$, 196.8 g of nickel nitrate $[Ni(NO_3)_2\cdot6H_2O]$, 225.1 g of cobalt nitrate $[Co(NO_3)_2\cdot6H_2O]$, 65.88 g of cerium nitrate $[Ce(NO_3)_3\cdot6H_2O]$, and 3.077 g of rubidium nitrate $[RbNO_3]$ in 400 g of nitric acid the concentration of which is 16.6% by mass was added in the stirring and mixing vessel to obtain an aqueous raw material mixture (starting material slurry) (first step).

Next, the aqueous raw material mixture was spray-dried using a spraying apparatus provided with a dish type rotor installed at the center of the upper portion of a dryer under a condition of an inlet temperature of about 230°C and an outlet temperature of about 120°C to obtain a dried particle (second step).

Subsequently, the dried particle was preliminarily calcined using an electric furnace at 320°C for 2 hours under an air atmosphere and was then finally calcined at 620°C for 2 hours under an air atmosphere (third step) to finally obtain 835 g of a catalyst.

The results that the obtained catalyst had a solid sphere shape, an average particle diameter of 53 $\mu$m, and an apparent specific gravity of 1.00 g/cc were obtained. Next, ammoxidation reaction of propylene was performed using 50 g of the catalyst at a contact time of $\Theta$ = 3.8 (sec·g/cc). The conversion ratio of propylene was 99.2%, the acrylonitrile (written as "AN" in Table 2) selectivity was 83.5%, and the acrylonitrile yield was 82.8% at 24 hours after the start of the reaction.

In addition, the attrition strength was measured for 50 g of the catalyst in accordance with the ACC method to find that the attrition loss (%) showed a low value, as low as 0.2%.

Further, when the sample after the attrition strength test was analyzed by X-ray diffraction, it was ascertained that the crystal phase of nickel molybdate was the $\beta$ type crystal phase, and the $\alpha$ type crystal phase was hardly present.

The catalyst composition (metal composition, the amount of silica ($SiO_2$) carrier, values of $\alpha$, $\beta$, $\gamma$, $\delta$) is shown in Table 1, and the reaction results and the results of physical property measurement are shown in Table 2.

[Comparative Example 11]

The reaction performance after 300 Hr was checked by continuing the ammoxidation reaction of propylene in Comparative Example 10 while finely adjusting the contact time so that the conversion ratio of propylene was maintained as it was to find that the contact time was deteriorated to $\Theta$ = 4.5 (sec·g/cc), and the acrylonitrile (written as "AN" in Table 2) selectivity was 83.1% and the acrylonitrile yield was 82.5% at a conversion ratio of propylene of 99.3%, and thus it was observed that these values were lowered. The reaction results are shown in Table 2.

[Table 1]

| | Mo | Bi | Fe | Ni | Mg | Co | Ce | Cr | Zn | X | SiO2 Amount of SiO2 carrier wt% | $\alpha$ | $\beta$ | $\gamma$ | $\delta$ | $\varepsilon$ |
|---|---|---|---|---|---|---|---|---|---|---|---|---|---|---|---|---|
| Examples 1, 2 ( * ) | 12 | 0.25 | 1.5 | 4.0 | 2.3 | 2.2 | 0.30 | 0 | 0 | Rb0.12 | 40 | 0.035 | 0.26 | 0.35 | 0.50 | 1.20 |
| Examples 3, 4 | 12 | 0.45 | 1.4 | 3.5 | 2.3 | 2.5 | 0.40 | 0 | 0 | Rb0.13 | 40 | 0.065 | 0.25 | 0.43 | 0.36 | 0.89 |
| Examples 3, 4 Examples 5, 6 | 12 | 0.70 | 0.9 | 1.0 | 2.3 | 5.0 | 0.45 | 0 | 0 | Rb0.12 | 45 | 0.109 | 0.16 | 1.52 | 0.64 | 0.64 |
| Examples 5, 6 Examples 7, 8 | 12 | 0.70 | 0.9 | 1.5 | 0.6 | 7.0 | 0.70 | 0 | 0 | Rb0.14 | 40 | 0.101 | 0.15 | 3.38 | -0.50 | 1.00 |
| Examples 9, 10 | 12 | 0.50 | 2.0 | 0.8 | 1.5 | 5.0 | 0.40 | 0 | 0 | Rb0.12 | 40 | 0.073 | 0.41 | 2.17 | 0.35 | 0.80 |
| Examples 11, 12 | 12 | 0.60 | 0.8 | 1.2 | 1.0 | 6.0 | 0.90 | 0 | 0 | K0.5Cs0.1 | 40 | 0.096 | 0.15 | 2.72 | 0.34 | 1.50 |
| Examples 11, 12 Examples 13, 14 | 12 | 0.35 | 1.0 | 1.2 | 1.0 | 5.5 | 0.90 | 0 | 0.5 | Rb0.12 | 40 | 0.054 | 0.18 | 2.03 | 0.42 | 2.57 |
| Examples 15, 16 | 12 | 0.28 | 2.1 | 1.0 | 0.4 | 5.2 | 0.75 | 0 | 0 | Rb0.16 | 40 | 0.043 | 0.48 | 3.71 | 0.70 | 2.68 |
| Examples 17, 18 | 12 | 0.40 | 2.3 | 1.8 | 0.3 | 3.5 | 0.75 | 0 | 0 | Rb0.15 | 37 | 0.066 | 0.62 | 1.67 | 1.23 | 1.88 |
| Examples 1, 2 | 12 | 0.40 | 0.6 | 5.8 | 0 | 3.4 | 0.20 | 0 | 0 | Rb0.10 | 40 | 0.059 | 0.10 | 0.59 | 1.00 | 0.50 |
| Comparative Examples 1, 2 Comparative Example 3 | 12 | 0.55 | 2.0 | 7.0 | 0 | 1.0 | 0.30 | 0 | 0 | Rb0.11 | 40 | 0.075 | 0.38 | 0.14 | -0.27 | 0.55 |
| Comparative Example 4 | 12 | 0.90 | 1.6 | 3.0 | 0.8 | 3.8 | 0.60 | 0 | 0 | Rb0.15 | 40 | 0.135 | 0.32 | 1.00 | -0.25 | 0.67 |
| Comparative Example 5 | 12 | 0.40 | 2.0 | 0.3 | 0.8 | 5.5 | 0.90 | 0 | 0 | Rb0.10 | 40 | 0.063 | 0.45 | 5.00 | 0.45 | 2.25 |
| Comparative Example 6 | 12 | 0.15 | 1.6 | 4.5 | 1.0 | 2.8 | 0.60 | 0 | 0 | Rb0.13 | 40 | 0.021 | 0.29 | 0.51 | 0.18 | 4.00 |
| Comparative Example 7 | 12 | 0.40 | 3.5 | 2.9 | 2.3 | 2.0 | 0.30 | 0 | 0 | Rb0.10 | 40 | 0.048 | 0.73 | 0.38 | -1.50 | 0.75 |
| Comparative Examples 8, 9 | 12 | 0.25 | 1.4 | 4.C | 2.3 | 3.0 | 0.70 | 0.2 | 0 | Rb0.10 | 40 | 0.032 | 0.26 | 0.48 | -1.13 | 2.80 |
| Comparative Examples 10, 11 | 12 | 0.35 | 1.6 | 3.5 | 0 | 4 | 0.80 | 0 | 0 | Rb0.11 | 50 | 0.053 | 0.32 | 1.14 | 0.38 | 2.29 |

(*) Examples 1,2 are not according to the presently claimed invention

[Table 2]

| | Elapsed time of Reaction (Hr) | Reaction conditions and results | | | | | Results of physical property measurement | | |
|---|---|---|---|---|---|---|---|---|---|
| | | $\Theta$ (sec·g/cc) | Starting material composition (molar ratio) C3H6/NH3/Air | Conversion ratio of propylene (%) | AN selectivity (%) | AN yield (%) | Average particle diameter $\mu$m | Attrition strength (%) | Apparent specific gravity g/cc |
| Example 1( * ) | 24 | 4.3 | 1/1.28/9.05 | 99.2 | 85.8 | 85.1 | 56 | 1.0 | 0.97 |
| Example 2( * ) | 300 | 4.5 | 1/1.21/8.86 | 99.1 | 85.6 | 84.8 | - | - | - |
| Example 3 | 24 | 4.2 | 1/1.29/9.16 | 99.3 | 85.0 | 84.4 | 58 | 1.2 | 1.00 |
| Example 4 | 300 | 4.5 | 1/1.22/9.01 | 99.3 | 84.8 | 84.2 | - | - | - |
| Example 5 | 24 | 3.6 | 1/1.18/8.81 | 99.1 | 85.6 | 84.8 | 54 | 0.4 | 0.98 |
| Example 6 | 300 | 3.6 | 1/1.14/8.73 | 99.2 | 85.4 | 84.7 | - | - | - |
| Example 7 | 24 | 3.8 | 1/1.17/8.81 | 99.2 | 84.9 | 84.2 | 55 | 0.7 | 1.01 |
| Example 8 | 300 | 3.9 | 1/1.15/8.80 | 99.2 | 84.9 | 84.2 | - | - | - |
| Example 9 | 24 | 3.6 | 1/1.27/9.24 | 99.2 | 85.2 | 84.5 | 52 | 0.8 | 1.01 |
| Example 10 | 300 | 3.8 | 1/1.20/8.96 | 99.1 | 85.3 | 84.5 | - | - | - |
| Example 11 | 24 | 4.0 | 1/1.17/8.78 | 99.3 | 85.5 | 84.9 | 53 | 0.6 | 1.04 |
| Example 12 | 300 | 4.1 | 1/1.13/8.71 | 99.3 | 85.4 | 84.8 | - | - | - |
| Example 13 | 24 | 3.7 | 1/1.22/8.98 | 99.3 | 85.2 | 84.6 | 52 | 0.4 | 1.02 |
| Example 14 | 300 | 3.8 | 1/1.18/8.90 | 99.2 | 85.1 | 84.4 | - | - | - |
| Example 15 | 24 | 4.0 | 1/1.26/9.18 | 99.2 | 85.1 | 84.4 | 51 | 0.4 | 1.01 |
| Example 16 | 300 | 4.0 | 1/1.20/8.94 | 99.2 | 85.3 | 84.6 | - | - | - |
| Example 17 | 24 | 3.9 | 1/1.26/9.23 | 99.3 | 84.7 | 84.1 | 54 | 0.5 | 1.02 |
| Example 18 | 300 | 4.0 | 1/1.21/9.05 | 99.2 | 84.9 | 84.2 | - | - | - |
| Comparative Example 1 | 24 | 4.3 | 1/1.30/9.51 | 99.0 | 83.8 | 83.0 | 53 | 0.3 | 1.02 |
| Comparative Example 2 | 300 | 4.8 | 1/1.19/9.02 | 99.2 | 83.2 | 82.5 | - | - | - |
| Comparative Example 3 | 24 | 4.1 | 1/1.24/9.31 | 99.2 | 83.1 | 82.4 | 54 | 0.4 | 1.02 |

| | Elapsed time of Reaction (Hr) | Reaction conditions and results | | | | | Results of physical property measurement | | |
|---|---|---|---|---|---|---|---|---|---|
| | | Θ (sec·g/cc) | Starting material composition (molar ratio) C3H6/NH3/Air | Conversion ratio of propylene (%) | AN selectivity (%) | AN yield (%) | Average particle diameter μm | Attrition strength (%) | Apparent specific gravity g/cc |
| Comparative Example 4 | 24 | 3.9 | 1/1.20/8.98 | 99.2 | 83.6 | 82.9 | 55 | 0.9 | 1.01 |
| Comparative Example 5 | 24 | 4.2 | 1/1.28/9.34 | 99.2 | 82.9 | 82.2 | 53 | 1.7 | 0.98 |
| Comparative Example 6 | 24 | 4.1 | 1/1.16/8.85 | 99.1 | 83.6 | 82.8 | 54 | 1.1 | 1.00 |
| Comparative Example 7 | 24 | 4.1 | 1/1.21/9.10 | 99.1 | 82.9 | 82.2 | 53 | 1.5 | 0.99 |
| Comparative Example 8 | 24 | 4.0 | 1/1.18/8.95 | 99.1 | 82.8 | 82.1 | 51 | 0.3 | 0.98 |
| Comparative Example 9 | 300 | 4.7 | 1/1.14/8.85 | 99.0 | 82.5 | 81.7 | - | - | - |
| Comparative Example 10 | 24 | 3.8 | 1/1.20/8.95 | 99.2 | 83.5 | 82.8 | 53 | 0.2 | 1.00 |
| Comparative Example 11 | 300 | 4.5 | 1/1.15/8.75 | 99.3 | 83.1 | 82.5 | - | - | - |
| (*) Examples 1 and 2 are not according to the presently claimed invention | | | | | | | | | |

EP 3 409 357 B1

29

The atomic ratio composition of each element in Table 1 is shown as a charge ratio in designing a catalyst. In addition, the amount of $SiO_2$ carrier (% by mass) in Table 1 represents the mass ratio of silica in the total mass of the catalyst after the nitrates and the ammonium salts, which are used as starting materials for the catalyst, and the organic acids, water, and the like are decomposed and evaporated in air at a calcination temperature of 500°C or higher to make the catalyst, and this is also shown as a value obtained as a charge ratio. As is clear from the results in Tables 1 to 2, when the catalyst for the fluidized bed ammoxidation reaction according to the present embodiment (Examples 3 to 18) is used, acrylonitrile can be produced at a high yield and stably over a long time even under conditions in which an excessive amount of ammonia based on propylene is not used in producing acrylonitrile through ammoxidation reaction of propylene. Moreover, it was understood that when the silica sol having an average particle diameter of primary particles in a range from 5 to 50 nm and having a standard deviation of less than 30% based on the average particle diameter in the particle diameter distribution of the primary particles is used as a starting material for silica, the attrition strength can be improved without lowering the yield of acrylonitrile, and therefore the catalyst is suitable for use for a further long time. It is to be noted that in Example 5, the silica sol having a large standard deviation is used and it is understood that a sufficient strength can be secured when the content of silica is made large. In addition, in Comparative Example 6, the ratio of the standard deviation to the average particle diameter of silica is small, as small as 5%, and it is inferred that the shape of the catalyst was distorted due to an influence of the catalyst composition (particularly, values of f and $\gamma$ in formula (1)) to lower the attrition strength.

Industrial Applicability

The catalyst for the fluidized bed ammoxidation reaction according to the present invention produces an aimed product at a high yield even under conditions in which an excessive amount of ammonia based on propylene is not used, and has excellent handling properties, such as the attrition resistance, the apparent specific gravity, and the particle diameter, as a fluidized bed catalyst in the case where the catalyst is used industrially. When ammoxidation reaction of propylene is performed in a fluidized bed reactor using the catalyst for the fluidized bed ammoxidation reaction according to the present invention, acrylonitrile can be produced at a high yield and therefore the catalyst for the fluidized bed ammoxidation reaction according to the present invention is industrially advantageous.

**Claims**

1. A catalyst for a fluidized bed ammoxidation reaction comprising:

   silica and
   a metal oxide, wherein
   a composite of the silica and the metal oxide is represented by the following formula (1):

   $$Mo_{12}Bi_aFe_bNi_cMg_dCO_eCe_fCr_gZn_hX_iO_j/(SiO_2)_A \qquad (1);$$

   wherein Mo represents molybdenum, Bi represents bismuth, Fe represents iron, Ni represents nickel, Mg represents magnesium, Co represents cobalt, Ce represents cerium, Cr represents chromium, Zn represents zinc, X represents at least one element selected from the group consisting of potassium, rubidium, and cesium, $SiO_2$ represents silica, a, b, c, d, e, f, g, h, i, and j each represent an atomic ratio of each element and satisfy $0.1 \leq a \leq 1$, $0.5 \leq b \leq 4$, $0.5 \leq c \leq 6.5$, $0.2 \leq d \leq 2.5$, $0.5 \leq e \leq 7$, $0.4 \leq f \leq 1.2$, $g \leq 0.05$, $h \leq 4$, and $0.05 \leq i \leq 1$, provided that j represents an atomic ratio of an oxygen atom, the atomic ratio satisfying valences of constituent elements excluding silica, A represents a content of silica (% by mass) in the composite and satisfies $35 \leq A \leq 48$, and values of $\alpha$, $\beta$, and $\gamma$ calculated from the atomic ratios of respective elements by the following expressions (2), (3), and (4) satisfy $0.03 \leq \alpha \leq 0.12$, $0.1 \leq \beta \leq 0.7$, and $0.2 \leq \gamma \leq 4$:

   $$\alpha = 1.5a/(1.5(b + g) + c + d + e + h) \qquad (2);$$

   $$\beta = 1.5(b + g)/(c + d + e + h) \qquad (3);$$

   and

$$\gamma = e/(c + d + h) \quad (4).$$

**2.** The catalyst for the fluidized bed ammoxidation reaction according to claim 1, wherein $\delta$ calculated from the atomic ratio of each element by the following expression (5) satisfies $-1 \leq \delta \leq 1.5$:

$$\delta = 12 - 1.5(a + b + f + g) - c - d - e - h \quad (5).$$

**3.** The catalyst for the fluidized bed ammoxidation reaction according to claim 1 or 2, wherein the X represents rubidium.

**4.** The catalyst for the fluidized bed ammoxidation reaction according to any one of claims 1 to 3, wherein $\varepsilon$ calculated from the atomic ratio of each element by the following expression (6) satisfies $0.5 \leq \varepsilon \leq 3.0$:

$$\varepsilon = f/a \quad (6).$$

**5.** A method for producing the catalyst for the fluidized bed ammoxidation reaction according to any one of claims 1 to 4, the method comprising:

a first step of preparing a starting material slurry;
a second step of obtaining a dried particle by spray-drying the starting material slurry; and
a third step of calcining the dried particle, wherein
a silica sol, which has an average particle diameter of primary particles of 5 nm or more and less than 50 nm and has a standard deviation of less than 30% based on the average particle diameter in a particle size distribution of the primary particles, is used as a starting material for the silica in the first step,
wherein the average particle diameter is determined as described in the description.

**6.** A method for producing acrylonitrile using the catalyst for the fluidized bed ammoxidation reaction according to any one of claims 1 to 4.


**Patentansprüche**

**1.** Katalysator für eine Wirbelschicht-Ammoxidierungsreaktion, umfassend:

Siliciumdioxid und
ein Metalloxid, wobei
ein Verbundstoff aus dem Siliciumdioxid und dem Metalloxid durch die folgende Formel (1) dargestellt wird:

$$Mo_{12}Bi_aFe_bNi_cMg_dCo_eCe_fCr_gZn_hX_iO_j/(SiO_2)_A \quad (1);$$

wobei Mo für Molybdän steht, Bi für Bismut steht, Fe für Eisen steht, Ni für Nickel steht, Mg für Magnesium steht, Co für Cobalt steht, Ce für Cer steht, Cr für Chrom steht, Zn für Zink steht, X für wenigstens ein Element steht, das aus der Gruppe ausgewählt ist, die aus Kalium, Rubidium und Cäsium besteht, $SiO_2$ für Siliciumdioxid steht, a, b, c, d, e, f, g, h, i und j jeweils für einen Atomanteil des jeweiligen Elements steht und die folgenden Beziehungen erfüllen: $0,1 \leq a \leq 1$, $0,5 \leq b \leq 4$, $0,5 \leq c \leq 6,5$, $0,2 \leq d \leq 2,5$, $0,5 \leq e \leq 7$, $0,4 \leq f \leq 1,2$, $g \leq 0,05$, $h \leq 4$, und $0,05 \leq i \leq 1$, mit der Maßgabe, dass j für einen Atomanteil an Sauerstoffatomen steht, wobei der Atomanteil die Valenzen der konstituierenden Elemente außer Siliciumdioxid absättigt, A für einen Gehalt an Siliciumdioxid (Massen-%) in dem Verbundstoff steht und die Bedingung $35 \leq A \leq 48$ erfüllt und Werte von $\alpha$, $\beta$ und $\gamma$, die anhand der Atomanteile der jeweiligen Elemente durch die folgenden Ausdrücke (2), (3) und (4) berechnet werden, die folgenden Bedingungen erfüllen: $0,03 \leq \alpha \leq 0,12$, $0,1 \leq \beta \leq 0,7$, und $0,2 \leq \gamma \leq 4$:

$$\alpha = 1,5a/(1,5(b + g) + c + d + e + h) \quad (2);$$

$$\beta = 1,5(b + g)/(c + d + e + h) \quad (3);$$

und

$$\gamma = e/(c + d + h) \qquad (4).$$

2. Katalysator für die Wirbelschicht-Ammoxidierungsreaktion gemäß Anspruch 1, wobei $\delta$, das aus dem Atomanteil jedes Elements durch den folgenden Ausdruck (5) berechnet wird, die Bedingung $-1 \leq \delta \leq 1{,}5$ erfüllt:

$$\delta = 12 - 1{,}5(a + b + f + g) - c - d - e - h \qquad (5).$$

3. Katalysator für die Wirbelschicht-Ammoxidierungsreaktion gemäß Anspruch 1 oder 2, wobei das X für Rubidium steht.

4. Katalysator für die Wirbelschicht-Ammoxidierungsreaktion gemäß einem der Ansprüche 1 bis 3, wobei $\varepsilon$, das aus dem Atomanteil jedes Elements durch den folgenden Ausdruck (6) berechnet wird, die Bedingung $0{,}5 \leq \varepsilon \leq 3{,}0$ erfüllt:

$$\varepsilon = f/a \qquad (6).$$

5. Verfahren zur Herstellung des Katalysators für die Wirbelschicht-Ammoxidierungsreaktion gemäß einem der Ansprüche 1 bis 4, wobei das Verfahren umfasst:

   einen ersten Schritt des Herstellens einer Ausgangsstoff-Aufschlämmung;
   einen zweiten Schritt des Gewinnens eines getrockneten Teilchens durch Sprühtrocknen der Ausgangsstoff-Aufschlämmung; und
   einen dritten Schritt des Calcinierens des getrockneten Teilchens, wobei
   ein Silicasol, das einen mittleren Teilchendurchmesser von Primärteilchen von 5 nm oder mehr und weniger als 50 nm aufweist und eine Standardabweichung von weniger als 30% aufweist, bezogen auf den mittleren Teilchendurchmesser in einer Teilchengrößenverteilung der Primärteilchen, als Ausgangsstoff für das Silica im ersten Schritt verwendet wird,
   wobei der mittlere Teilchendurchmesser so bestimmt wird, wie es in der Beschreibung beschrieben ist.

6. Verfahren zur Herstellung von Acrylnitril unter Verwendung des Katalysators für die Wirbelschicht-Ammoxidierungsreaktion gemäß einem der Ansprüche 1 bis 4.


**Revendications**

1. Catalyseur pour une réaction d'ammoxydation en lit fluidisé comprenant :

   de la silice et
   un oxyde de métal, dans lequel
   un composite de la silice et de l'oxyde de métal est représenté par la formule (1) suivante :

$$Mo_{12}Bi_aFe_bNi_cMg_dCo_eCe_fCr_gZn_hX_iO_j/(SiO_2)_A \qquad (1) ;$$

   où Mo représente le molybdène, Bi représente le bismuth, Fe représente le fer, Ni représente le nickel, Mg représente le magnésium, Co représente le cobalt, Ce représente le cérium, Cr représente le chrome, Zn représente le zinc, X représente au moins un élément choisi dans le groupe consistant en potassium, rubidium, et césium, $SiO_2$ représente la silice, a, b, c, d, e, f, g, h, i, et j représentent chacun un rapport atomique de chaque élément et satisfont $0{,}1 \leq a \leq 1$, $0{,}5 \leq b \leq 4$, $0{,}5 \leq c \leq 6{,}5$, $0{,}2 \leq d \leq 2{,}5$, $0{,}5 \leq e \leq 7$, $0{,}4 \leq f \leq 1{,}2$, $g \leq 0{,}05$, $h \leq 4$, et $0{,}05 \leq i \leq 1$, à condition que j représente un rapport atomique d'un atome d'oxygène, le rapport atomique satisfaisant les valences d'éléments constituants excluant la silice, A représente une teneur en silice (% en masse) dans le composite et satisfait $35 \leq A \leq 48$, et les valeurs de $\alpha$, $\beta$, et $\gamma$ calculées à partir des rapports atomiques d'éléments respectifs par les expressions (2), (3), et (4) suivantes satisfont $0{,}03 \leq \alpha \leq 0{,}12$, $0{,}1 \leq \beta \leq 0{,}7$, et $0{,}2 \leq \gamma \leq 4$ :

$$\alpha = 1{,}5a/(1{,}5(b + g) + c + d + e + h) \qquad (2) \; ;$$

$$\beta = 1{,}5(b + g)/(c + d + e + h) \qquad (3) \; ;$$

et

$$\gamma = e/(c + d + h) \qquad (4).$$

2. Catalyseur pour la réaction d'ammoxydation en lit fluidisé selon la revendication 1, dans lequel $\delta$ calculé à partir du rapport atomique de chaque élément par l'expression (5) suivante satisfait $-1 \leq \delta \leq 1{,}5$ :

$$\delta = 12 - 1{,}5(a + b + f + g) - c - d - e - h \qquad (5).$$

3. Catalyseur pour la réaction d'ammoxydation en lit fluidisé selon la revendication 1 ou 2, dans lequel le X représente le rubidium.

4. Catalyseur pour la réaction d'ammoxydation en lit fluidisé selon l'une quelconque des revendications 1 à 3, dans lequel $\varepsilon$ calculé à partir du rapport atomique de chaque élément par l'expression (6) suivante satisfait $0{,}5 \leq \varepsilon \leq 3{,}0$ :

$$\varepsilon = f/a \qquad (6).$$

5. Procédé pour la production du catalyseur pour la réaction d'ammoxydation en lit fluidisé selon l'une quelconque des revendications 1 à 4, le procédé comprenant :

une première étape de préparation d'une suspension de matière de départ ;
une seconde étape d'obtention d'une particule séchée par séchage par pulvérisation de la suspension de matière de départ ; et
une troisième étape de calcination de la particule séchée, dans lequel
un sol de silice, qui présente un diamètre moyen de particule de particules primaires de 5 nm ou supérieur et inférieur à 50 nm et présente un écart-type inférieur à 30 % sur la base du diamètre moyen de particule dans une distribution de taille de particule des particules primaires, est utilisé comme une matière de départ pour la silice dans la première étape,
dans lequel le diamètre moyen de particule est déterminé comme décrit dans la description.

6. Procédé pour la production d'acrylonitrile utilisant le catalyseur pour la réaction d'ammoxydation en lit fluidisé selon l'une quelconque des revendications 1 à 4.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010172851 A
- JP 5188005 B
- JP 4709549 B
- JP 4588533 B
- JP 5011167 B
- JP 5491037 B
- JP 2013017917 A
- JP 2013169482 A
- WO 2013522038 A